# EUROPÄISCHE PATENTSCHRIFT

(11) **EP 2 510 892 B1**
(45) Veröffentlichungstag und Bekanntmachung des Hinweises auf die Patenterteilung: **04.12.2019**
(21) Anmeldenummer: 12161972.0
(22) Anmeldetag: 29.03.2012
(51) Int. Cl.: A61B 17/70, A61B 17/86, A61B 17/02, A61B 17/17, A61B 90/00, A61B 17/88

(54) **Chirurgisches Distraktionsinstrument für die Laminoplastie**
Surgical distraction instrument for laminoplasty
Instrument d'expansion chirurgical pour la laminoplastie

(30) Priorität: 12.04.2011 DE 102011001997
(43) Veröffentlichungstag der Anmeldung: 17.10.2012
(73) Patentinhaber: AESCULAP AG, 78532 Tuttlingen (DE)
(72) Erfinder: Beger, Jens, 78532 Tuttlingen (DE); Linke, Ralph, 78256 Steisslingen (DE); Suchomel, Petr, 460063 Liberec (CZ)
(74) Vertreter: Hoeger, Stellrecht & Partner Patentanwälte mbB

(56) Entgegenhaltungen:
- EP-A1- 2 196 160
- DE-U1-202010 000 341
- US-A1- 2006 074 431
- US-A1- 2009 177 285
- US-A1- 2010 241 165

## Beschreibung

Die Erfindung betrifft ein chirurgisches Instrumentarium mit einem Distraktionsinstrument sowie ein Halteinstrument für ein einzusetzendes Implantat zur Verwendung in der Laminoplastie, bei der der Wirbelbogen eines Wirbels unter Bildung eines Schnittspalts durchtrennt und der Schnittspalt zum Einsetzen eines Implantats mit dem Distraktionsinstrument aufgeweitet wird. Die Wirbelkanäle der Wirbel der Wirbelsäule bilden den so genannten Rückenmarks- oder Spinalkanal, in dem das Rückenmark, umhüllt von der Rückenmarkshaut, aufgenommen ist.

Das Rückenmark als Teil des zentralen Nervensystems kann in seiner Funktion beeinträchtigt werden, wenn z.B. beim Vorliegen einer Spinalkanalstenose Druck auf das Rückenmark ausgeübt wird, was mehrere Ursachen haben kann, beispielsweise das Vorliegen einer Spondylose oder einer Verknöcherung des hinteren Längsbandes.

Abhilfe geschaffen werden kann hier, indem der Wirbelkanal des oder der betroffenen Wirbel der Wirbelsäule vergrößert wird, so dass das Rückenmark mehr Raum zur Verfügung hat und somit dem Druck ausweichen kann.

Eine Übersicht über die bislang gängigen Therapiemöglichkeiten findet sich z.B. bei F. Meyer et al., Deutsches Ärzteblatt, Jg. 105, Heft 20, Seiten 366 bis 372. Neben den ventralen Verfahren kommen verschiedene dorsale Verfahren, nämlich die Laminektomie mit und ohne Fusion sowie die Laminoplastie, zum Einsatz. Gegebenenfalls können ventrale auch mit dorsalen Verfahren kombiniert zum Einsatz kommen.

Von den verschiedenen dorsalen Verfahren kommt die Laminoplastie mit den geringfügigsten Eingriffen in die Knochensubstanz aus.

Vorgeschlagen wurden bisher verschiedene Laminoplastie-Operationstechniken, von denen die beiden wichtigsten in der Literatur als so genannte Single-Door- oder Double-Door-Techniken beschrieben werden. Eine Übersicht hierüber und eine Bewertung der zu erwartenden Effekte bezüglich der Druckentlastung bzw. der Expansion des Spinalkanals ist beispielsweise in der Veröffentlichung von Wang, Xiang-Yang et al. in SPINE, Vol. 31, Nr. 24, 2006, Seiten 2863 bis 2870, enthalten.

Bei der sogenannten Single-Door-Technik, auch Open-Door-Technik genannt, wird die Lamina auf einer Seite des Wirbels mit einem Schnittspalt durchtrennt, während auf der anderen Seite der Lamina eine Kerbe ohne Durchtrennung des Wirbelbogens geschaffen wird.

Der Bereich des Wirbelbogens mit der Einkerbung fungiert bei der nachfolgenden Eröffnung des Wirbelkanals als eine Art Scharnier und erlaubt ein Öffnen des Wirbelbogens, das mit einem Bruch der Knochensubstanz verbunden ist. Der Wirbelbogen bleibt über die Knochenhaut und die Kollagenfasern der Knochensubstanz mit dem Wirbelkörper verbunden.

Bei der sogenannten Double-Door-Technik wird der Dornfortsatz eines Wirbels durchtrennt oder ganz entfernt und beidseits des Dornfortsatzes in der Lamina jeweils eine Kerbe gesetzt, wobei die die Kerben enthaltenden Bereiche des Wirbelbogens wieder als Scharniere fungieren. Die Eröffnung des Wirbelkanals erfolgt nun durch das Auseinanderschwenken der beiden Wirbelbogenabschnitte mit den gegebenenfalls noch vorhandenen zugehörigen Dornfortsatzteilen, wobei ebenfalls die Knochensubstanz im Bereich der Scharniere bricht. Die Wirbelbogenabschnitte bleiben auch hier über die Knochenhaut und die Kollagenfasern der Knochensubstanz mit dem Wirbelkörper verbunden.

Der Wirbelkanal der Wirbel wird bei beiden Techniken im eröffneten Zustand durch Implantate fixiert. Als Implantatmaterial kommt neben körpereigenem Knochenspan ein Hydroxyapatitspacer oder dgl. zum Einsatz.

Trotz des gegenüber anderen dorsalen Verfahren verminderten Eingriffs in die Knochensubstanz wird bei der Laminoplastie eine deutlich erhöhte Rate von anschließenden Nackenschmerzen noch als nachteilig empfunden, ebenso wie eine oft beobachtete Einschränkung der Beweglichkeit der Halswirbelsäule. Im Stand der Technik ist es bekannt, chirurgische Instrumente zu verwenden, um den Abstand zweier benachbarter Wirbelkörper zu vergrößern. Beispielhaft werden hierzu die US 2009/0177285 A1, die EP 2 196 160 A1, die US 2006/0074431 A1 sowie die DE 20 2010 000 341 U1 genannt.

Aus der US 2010/0241165 A1 ist ein Distraktionsinstrument zum Erweitern eines Wirbelkanals eines Wirbels im Rahmen einer Laminoplastie bekannt.

Aufgabe der Erfindung ist es, ein chirurgisches Instrumentarium vorzuschlagen, mit dem eine Erweiterung des Wirbelkanals von Wirbeln mit geringeren Belastungen für den Patienten möglich ist.

Diese Aufgabe wird von einem chirurgischen Instrumentarium mit den Merkmalen des Anspruchs 1 gelöst.

Vorteilhafte Weiterbildungen der Erfindung sind Gegenstand der abhängigen Ansprüche.

Das erfindungsgemäße Instrumentarium wird vorzugsweise bei Verfahren verwendet, bei denen im Unterschied zu den zuvor diskutierten Single- und Double-Door-Techniken der Laminoplastie zum Einen nur noch ein einziger Trennschnitt am Wirbelbogen, insbesondere im Bereich des Dornfortsatzes, vorgenommen wird und so eine Ablösung von Muskulatur von der Wirbelsäule in großem Umfang oder sogar im Wesentlichen ganz vermieden wird. Die Aufweitung des Wirbelbogens wird mittels elastisch/plastischer Verformung desselben erzielt wird, so dass ein Brechen der Knochensubstanz der Lamina vermieden werden kann. Die offene Spreizstellung des Distraktionsinstruments wird auch als Distraktionsstellung bezeichnet.

Aufgrund der inhärenten viskoelastischen Eigenschaften der Knochensubstanz kommt es bei diesem Verfahren zusätzlich zu der elastisch/ plastischen Verformung, ohne dass dies jedoch zu einem Bruch der Knochensubstanz führt. Die Aufweitung wird bevorzugt nicht abrupt erzwungen, sondern allmählich ausgeführt, so dass die viskoelastischen Eigenschaften der Knochensubstanz zum Tragen kommen können. Dies kann kontinuierlich oder in kleinen Schritten von z.B. jeweils ca. 0,5 bis ca. 3 mm geschehen. Typischerweise lässt sich auf diese Weise eine Spaltaufweitung von ca. 10 bis ca. 12 mm innerhalb von ca. 10 sec bis ca. 5 min, insbesondere ca. 30 sec bis ca. 3 min, erzielen.

Diese elastisch/plastische Verformung zur Aufweitung des Schnittspalts bei minimiertem Risiko des Bruchs der Knochensubstanz lässt sich insbesondere dadurch erreichen, dass man die Kräfte, die beim Aufweiten eingesetzt werden, auf ca. 500 N oder weniger, insbesondere ca. 300 N oder weniger, limitiert.

Somit wird dem Problem der bisherigen Laminoplastietechniken entgegengewirkt, die zunächst zwingend weitreichende Eingriffe an der parallel zur Wirbelsäule verlaufenden Muskulatur notwendig machen, die auch in der nachoperativen Phase eine erhebliche Belastung für den Patienten bedeuten.

Der Schnittspalt kann mit sehr unterschiedlichen Werkzeugen erzeugt werden. Beispielsweise kann der Schnittspalt mit einem Ultraschall-Osteotom erzeugt werden, wobei Schnittspalte mit ca. 1 mm oder weniger resultieren.

Andere Techniken nutzen Hochgeschwindigkeitsbohrer oder Fräser (Craniotom), mit denen etwas breitere Schnittspalte erhalten werden, beispielsweise im Bereich von ca. 2 mm bis ca. 3 mm.

Noch eine andere Technik nutzt die sogenannte T-Säge oder Gigli-Säge, wobei hier eine gewisse Schwierigkeit in der anfänglichen Einführung der Säge ohne Verletzung der Rückenmarkshaut liegt. Ähnlich verhält es sich bei der Verwendung des Craniotoms.

Trotz der minimalen Eingriffe kann überraschenderweise mit der beschriebenen Laminoplastietechnik ein Zugang zum Spinalkanal bzw. dessen Erweiterung erzeugt werden, der hinsichtlich des zu erwartenden klinischen Ergebnisses vergleichbar ist mit dem Verfahren der herkömmlichen Single- und Double-Door-Technik.

Zur elastischen/plastischen Aufweitung des Schnittspalts werden die erfindungsgemäßen Distraktionsinstrumente, insbesondere in Form einer Distraktionszange, verwendet, welche vorzugsweise Werkzeugelemente aufweisen, die an ihrem distalen Ende eine Anlagefläche zur Positionierung des Instruments im Spalt aufweisen.

Die Anlageflächen sind insbesondere am distalen bzw. freien Ende der Werkzeugelemente als seitlich abstehende Flansche oder abgewinkelte Spitzen ausgebildet. Diese bevorzugten erfindungsgemäßen Distraktionsinstrumente werden in Sagittalrichtung in den Schnittspalt eingeführt, wobei die Anlageflächen der Werkzeugelemente auf der Spinalkanalseite der Wirbelbogenabschnitte zur Anlage kommen und so eine Führung für das Instrument bilden.

Die Anlageflächen sorgen ferner für einen sicheren Sitz des erfindungsgemäßen Distraktionsinstruments während der Aufweitung des Schnittspalts.

Beim Einführen des Distraktionsinstruments kann der Schnittspalt mit einer dünnen Klinge oder einem Raspatorium leicht geweitet werden.

Die Kräfte, die notwendig sind, um den Schnittspalt aufzuweiten, betragen typischerweise ca. 70 N bis ca. 200 N, insbesondere ca. 80 N bis ca. 150 N, für eine Aufweitung des Spalts um ca. 5 mm bis ca. 12 mm, jeweils bestimmt am Spinalkanal-seitigen Ende des Schnittspalts.

Die elastisch/plastische Aufweitung gelingt ganz überwiegend ohne Bruch des Knochenmaterials. Wenn ein Bruch beim Aufweiten um bis ca. 10 mm beobachtet wurde, dann betraf es den Bruch im Bereich des Dornfortsatzes.

Bevorzugt werden deshalb die Distraktionskräfte möglichst nahe zum oder besser im Bereich des Wirbelbogens und nicht im Bereich des Dornfortsatzes eingeleitet.

Vorzugsweise weisen die Werkzeugelemente am distalen Ende ein Anschlagselement auf, das der Positionierung des Implantats im Schnittspalt dient. Das Anschlagselement ist bevorzugt als nach vorn abstehender Flansch ausgebildet, welcher bei besonders bevorzugten Ausführungsformen direkt an die die Anlageflächen bildenden seitlich abstehenden Flansche anschließt.

Das Anschlagselement erlaubt ein sicheres Platzieren des Implantats im Schnittspalt, insbesondere kann es einem zu tiefen Einsetzen des Implantats vorbeugen, bei dem der ventrale Endbereich des Implantats in den Spinalkanal hineinragen würde.

Für den Chirurgen entsteht beim Einsetzen des Implantats, vorzugsweise entlang der Branchen des erfindungsgemäßen Distraktionsinstruments, ein haptisch deutlich wahrnehmbares Signal, wenn der ventrale Endbereich des Implantats auf das Anschlagselement trifft und anzeigt, dass die gewünschte Einsetztiefe für das Implantat erreicht ist.

Damit ist ein sicheres Platzieren des Implantats auch dann gesichert, wenn die Sicht auf den Schnittspalt beispielsweise durch Wundflüssigkeiten behindert ist.

Weiter bevorzugte erfindungsgemäße Distraktionsinstrumente weisen Werkzeugelemente auf, deren Querschnitt in dem in den Schnittspalt einzuführenden Endbereich keilförmig ausgebildet ist. Damit ist die Zentrierung des Instruments bei dessen Einführung in den Schnittspalt in Sagittalrichtung erleichtert. Auch lässt sich mit dieser Ausführungsform des erfindungsgemäßen Distraktionsinstruments das Werkzeug in Schnittspalte einführen, deren ursprüngliche Spaltbreite sehr gering ist, ohne auf weitere Hilfsmittel zurückgreifen zu müssen.

Diesem Zweck dient ein weiteres Merkmal eines erfindungsgemäßen Distraktionsinstruments, bei dem die Werkzeugelemente zu ihrem Ende in Richtung der Schließrichtung hin konisch verjüngt ausgebildet sind.

Um zu einer definierten Aufweitung des Schnittspalts zu kommen, weisen die Werkzeugelemente in Öffnungsrichtung eine Biegefestigkeit auf, die einer Distraktionskraft von ca. 300 N oder mehr entspricht, ohne dass dabei eine plastische Verformung an den Werkzeugelementen auftritt.

Für eine bessere Sicht auf den Schnittspalt sorgt die bevorzugte Maßnahme, die Längsachse der die Werkzeugelemente haltenden Branchen gegenüber der Längsachse oder Schwenkebene des Griffteils in einem stumpfen Winkel anzuordnen.

Der stumpfe Winkel wird vorzugsweise im Bereich von ca. 110 ° bis ca. 160 °, beispielsweise ca. 135 °, gewählt.

Die die Werkzeuge haltenden Branchen weisen vorzugsweise auf ihrer Oberseite, d.h. in der Richtung, in der auch die optional vorhandenen Anschlagselemente abstehen, Führungselemente auf, die ein geführtes Einsetzen des Implantats in den Schnittspalt ermöglichen.

Vorzugsweise weist das erfindungsgemäße Instrument eine Rastvorrichtung auf, die insbesondere am Griffelement montiert ist, mittels welcher die Werkzeugelemente in einer oder mehreren vorgegebenen Relativpositionen in Öffnungsrichtung zueinander festlegbar sind.

Damit ist für die stufenweise Aufweitung des Schnittspalts zum optimalen Ausnutzen der viskoelastischen Eigenschaften der Knochensubstanz des Wirbelbogens ein wichtiges Hilfsmittel gegeben.

Alternativ oder in Kombination mit dieser Weiterbildung des erfindungsgemäßen Distraktionsinstrumentes kann dieses mit einer Vorrichtung zur Bestimmung des Abstands der Endbereiche der Werkzeugelemente zueinander versehen sein. Damit lässt sich beispielsweise im Bereich des Griffteils die distal erreichte Spaltweite des Schnittspalts einfach ablesen.

Ergänzend oder alternativ zu den beiden zuvor besprochenen Maßnahmen kann vorgesehen sein, dass das erfindungsgemäße Distraktionsinstrument eine Kraftmess-Vorrichtung umfasst, so dass unter Vermeidung zu großer Kräfte die Aufweitung des Schnittspalts vorgenommen werden kann. Vorzugsweise weist die Kraftmess-Vorrichtung eine Anzeige für eine vorgegebene maximale, möglichst nicht zu überschreitende Kraft, beispielsweise von 300 N oder weniger auf.

Des Weiteren beinhaltet das erfindungsgemäße chirurgische Instrumentarium ein Halteinstrument für ein einzusetzendes Implantat, das es dem Chirurgen erleichtert, dieses in seine korrekte Position in dem Schnittspalt einzusetzen.

Ein bevorzugtes Halteinstrument weist ein proximal angeordnetes Griffteil, einen daran anschließenden Schaft und eine am distalen Ende des Schafts angeordnete Haltevorrichtung zum lösbaren Fixieren eines Implantats auf.

Bevorzugt wird die Haltevorrichtung einen Schlitten aufweisen, der komplementär zu Führungselementen des Distraktionselements ausgebildet ist oder der Führungsflächen aufweist, die mit den die Werkzeugelemente des Distraktionselements haltenden Branchen kooperieren und so ein geführtes Einsetzen des Implantats in den Schnittspalt erlauben.

Vor dem Einsetzen eines Implantats kann bedarfsweise auch vorübergehend ein Abstandhalter in den aufgeweiteten Schnittspalt eingesetzt werden, der ebenfalls Bestandteil eines bevorzugten erfindungsgemäßen Instrumentariums ist.

Der Abstandhalter weist vorzugsweise ein Abstandselement auf, welches vorzugsweise U-förmig ausgebildet ist und einen Zugang zum Rückenmarkskanal freihält. Auch hier ist es bevorzugt, den Abstandhalter so tief in den Schnittspalt einzuführen, dass er im Bereich des Wirbelbogens zur Anlage kommt und nicht oder nicht ausschließlich im Bereich des Dornfortsatzes. Die Orientierung der U-Form des Abstandselements zum Wirbel ist dabei cranial/caudal.

Der Abstandhalter wird oft bereits ausreichend durch die elastisch/plastisch gespreizten Wirbelbogen in dem Schnittspalt gehalten. Will man ein unbeabsichtigtes Bewegen des Abstandhalters vermeiden, kann dieser an der Haut des Patienten oder über einen optionalen Schaft an einem die Operationswunde offen haltenden Retraktor festgelegt werden, welcher optional ebenfalls Teil des erfindungsgemäßen Instrumentariums ist.

Der eingesetzte Abstandhalter mit U-förmigem Abstandselement erlaubt einen im Wesentlichen ungehinderten Blick und Zugang auf das Rückenmark bzw. die Rückenmarkshaut.

Die Aufweitung des Wirbelkanals eines Wirbels mit dem erfindungsgemäßen Instrumentarium schafft auch soweit einen Zugang zu cranial oder caudal benachbarten Wirbelkanälen, so dass dort mit der Technik des sogenannten Undercutting eine Dekompression des Rückenmarks erzielbar ist.

Beispielsweise kann so mittels dem erfindungsgemäßen Instrumentarium eine Aufweitung der Wirbelkanäle der C4- und C6-Wirbel eine Dekompression in dem gesamten Abschnitt der Halswirbelsäule von C3 bis C7 erzielt werden.

Bevorzugt werden die beiden Schnittflächen, die den Schnittspalt in dem Wirbelkörper definieren, auf einen Abstand von ca. 5 mm bis ca. 15 mm aufgeweitet, gemessen am Spinalkanal-seitigen Ende des Schnittspalts.

Die Schnittflächen des Schnittspalts werden in der aufgeweiteten Position über Instrumente oder Implantate fixiert, ähnlich wie dies auch im Zusammenhang mit der Operationsmethode im Stand der Technik möglich ist.

Das Material, aus dem das Implantat gefertigt wird, ist bevorzugt ein körperverträgliches Kunststoffmaterial, insbesondere PEEK, oder Titan oder eine Titanlegierung. Daneben eignen sich auch körpereigene Knochenspäne. Implantate aus Kunststoff, insbesondere PEEK, werden bevorzugt an den mit dem Knochenmaterial in Berührung stehenden Oberflächen mit einer osteointegrativen Beschichtung versehen. Vorzugsweise wird diese Beschichtung als mikroporöse Reintitanschicht nach dem VPS-Verfahren (Plasmapore-Technik) oder als Hydroxyapatit-Schicht ausgeführt.

Die Kunststoff-Implantate sind gegenüber Titan-Implantaten bevorzugt, da sie zum MRT-Verfahren kompatibel sind. Dies ist insbesondere für die postoperative Phase von Bedeutung. Die MRT-Kompatibilität ist auch bei den oben beschriebenen osteointegrativ-beschichteten Kunststoffimplantaten gegeben. Bevorzugt wird das Implantat eine Keilform aufweisen, so dass eine möglichst vollflächige Anlage der Oberflächen des keilförmigen Körpers an den Schnittflächen des aufgeweiteten Schnittspalts erzielbar ist, die nach der Bildung des Schnittspalts zunächst parallel, in Folge der elastischen Aufweitung des Schnittspalts aber keilförmig gegeneinander geneigt angeordnet sind.

Diese und weitere Vorteile der Erfindung werden im Folgenden anhand der Zeichnung noch näher erläutert. Es zeigen im Einzelnen:
- Figuren 1A und 1B: einen Teil der Halswirbelsäule mit einem mit einem Schnittspalt versehenen Halswirbel in perspektivischer bzw. Draufsicht;
- Figur 2A: eine erste Ausführungsform eines erfindungsgemäßen Distraktionsinstruments;
- Figur 2B: ein Detail des erfindungsgemäßen Distraktionsinstruments der Figur 2A;
- Figur 2C: die Darstellung des Halswirbels der Figur 1B bei der Aufweitung mit eingesetztem erfindungsgemäßem Distraktionsinstrument;
- Figur 3: eine schematische Schnittansicht durch einen erfindungsgemäß elastisch aufgeweiteten Wirbelkanal;
- Figuren 4A bis 4D: Gesamtdarstellung und Detailansichten einer weiteren Ausführungsform eines erfindungsgemäßen Distraktionsinstruments;
- Figuren 5A und 5B: Wirbel mit aufgeweitetem Schnittspalt und einem eingesetzten erfindungsgemäßen Abstandhalter;
- Figur 6A und 6B: eine perspektivische Darstellung eines alternativen Retraktors sowie eine Draufsicht auf einen aufgeweiteten Schnittspalt eines Wirbels entsprechend Figuren 5A und 5B mit dem alternativen Retraktor in Draufsicht;
- Figuren 7A und 7B: eine operative Behandlung im Wirbelkanal bei eingesetztem erfindungsgemäßem Abstandhalter im Schnittspalt;
- Figuren 8A und 8B: Grundformen einer ersten Ausführungsform eines Implantats in zwei Varianten;
- Figur 9: das Einsetzen eines Implantats in den von einem erfindungsgemäßen Abstandhalter offen gehaltenen Schnittspalt;
- Figur 10: das in dem Schnittspalt des Wirbels eingesetzte Implantat der Figur 8B;
- Figur 11A bis 11D: weitere Varianten des Implantats in perspektivischer und Schnitt-Darstellung;
- Figur 12A und 12B: Haltevorrichtung für ein Implantats im zusammengebauten und separierten Zustand;
- Figur 13A bis 13D: Details der Haltevorrichtung der Figur 12 in verschiedenen Darstellungen;
- Figur 14A bis 14C: die Haltevorrichtung der Figur 12 beim Einsetzen eines Implantats in einem Schnittspalt, der von einem erfindungsgemäßen Distraktionsinstrument der Figur 2 aufgeweitet ist;
- Figur 15: eine alternative erfindungsgemäße Haltevorrichtung für das Implantat;
- Figur 16A und 16B: eine alternative Ausführungsform des erfindungsgemäßen Abstandhalters der Figur 5; und
- Figur 17A und 17B: eine weiteres alternatives erfindungsgemäßes Distraktionsinstrument bei eingesetztem Implantat.

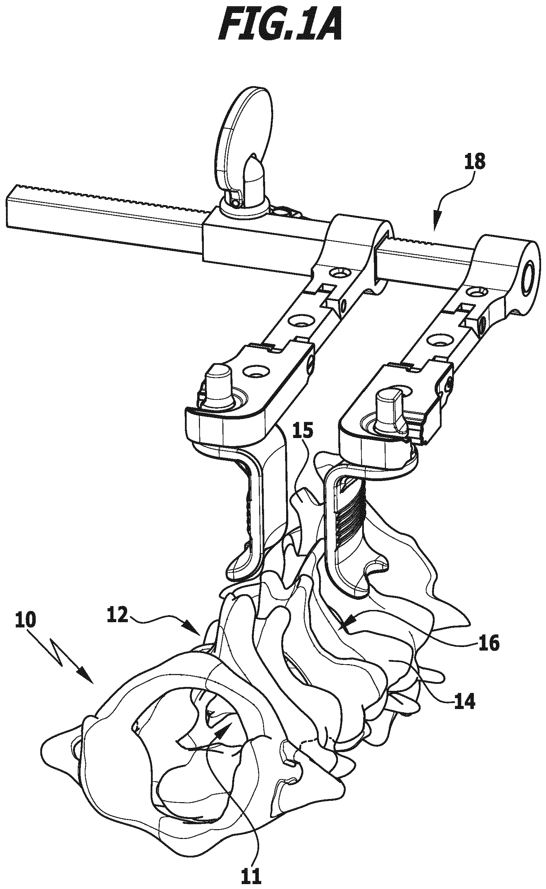

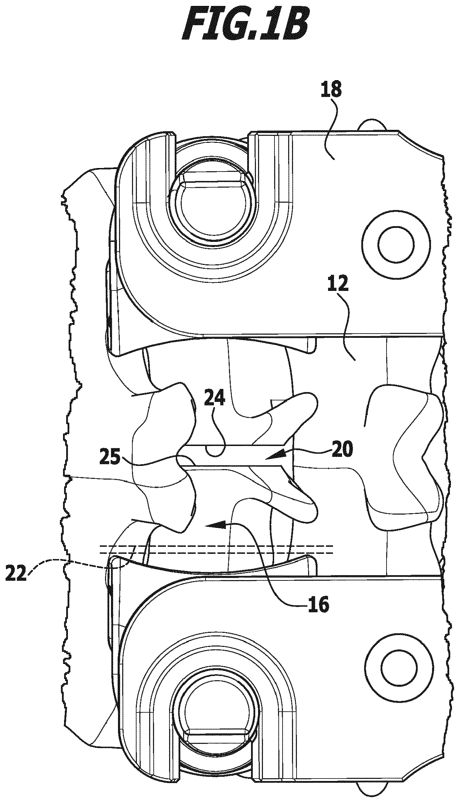

Figur 1A zeigt in schematischer Darstellung den Abschnitt C1 bis C7 einer Halswirbelsäule 10 mit einem Rückenmarkskanal 11 sowie einem über einem Wirbelbogen 12 eines Wirbels 14 positionierten Retraktor 18, der das umgebende (in der Figur 1 der Übersichtlichkeit halber weggelassene) Gewebe zurückhält, so dass der Bereich des Wirbels 14 dorsal zugänglich bleibt. Der Retraktor bildet einen Teil eines erfindungsgemäßen Instrumentariums.

Der Zugang zu dem Wirbel 14 und dessen Wirbelbogen 12 kann ohne Ablösung von Muskelgewebe geschehen und so dessen Dornfortsatz 15 zugänglich gemacht werden, während der Zugang zur Lamina 16 eine Ablösung von Muskelgewebe notwendig macht.

In dem in Figur 1A gezeigten Zustand kann mit einem Instrument (nicht gezeigt) im Bereich der Lamina 16 oder im Bereich des Dornfortsatzes 15 ein einziger Schnittspalt 20 oder 22 erzeugt werden, der eine elastisch/plastische Aufweitung des Wirbelbogens 12 bzw. seiner durch den Schnittspalt 20 oder 22 getrennten Wirbelbogenabschnitte ermöglicht, so dass weitere Bereiche des Wirbels nicht freigelegt werden müssen.

Die Herstellung des Schnittspalts ist nicht auf eine bestimmte Verfahrensweise limitiert. So kann der Schnittspalt z.B. mit einem Ultraschall-Osteotom hergestellt werden, das eine besonders schonende Durchtrennung der Knochensubstanz bis zum Rückenmarkskanal 11 erlaubt. Eine Schädigung des Bindegewebes des Rückenmarks wird hier vermieden.

Alternativ kann mit schnell drehenden Bohrern gearbeitet werden, wobei die letzte Phase des Trennschnitts bis zum Rückenmark vorzugsweise mit einer Knochenstanze durchgeführt wird.

Eine weitere Alternative bietet die sogenannte T- oder Gigli-Säge, mit der ausgehend vom Rückenmarkskanal 11 der Schnittspalt hergestellt wird.

Figur 1B zeigt den Wirbel 14 mit im Dornfortsatz fertiggestelltem Schnittspalt 20. Der alternative Schnittspalt 22 in einer der Lamina des Wirbelbogens 12 ist in durchbrochener Darstellung angedeutet.

Der Schnittspalt 20 weist zwei in dem in Figur 1B gezeigten Zustand parallel angeordnete Schnittflächen 24 und 25 auf. Die in diesem Zustand vorhandene Spaltbreite hängt von der zur Erzeugung des Schnittspalts eingesetzten Technik ab und beträgt beispielsweise bei Verwendung eines Ultraschall-Ostetoms ca. 1 mm oder weniger. Bei schnelldrehenden Bohrern wird typischerweise eine Breite des Schnittspalts von ca. 2 mm bis 3 mm erhalten.

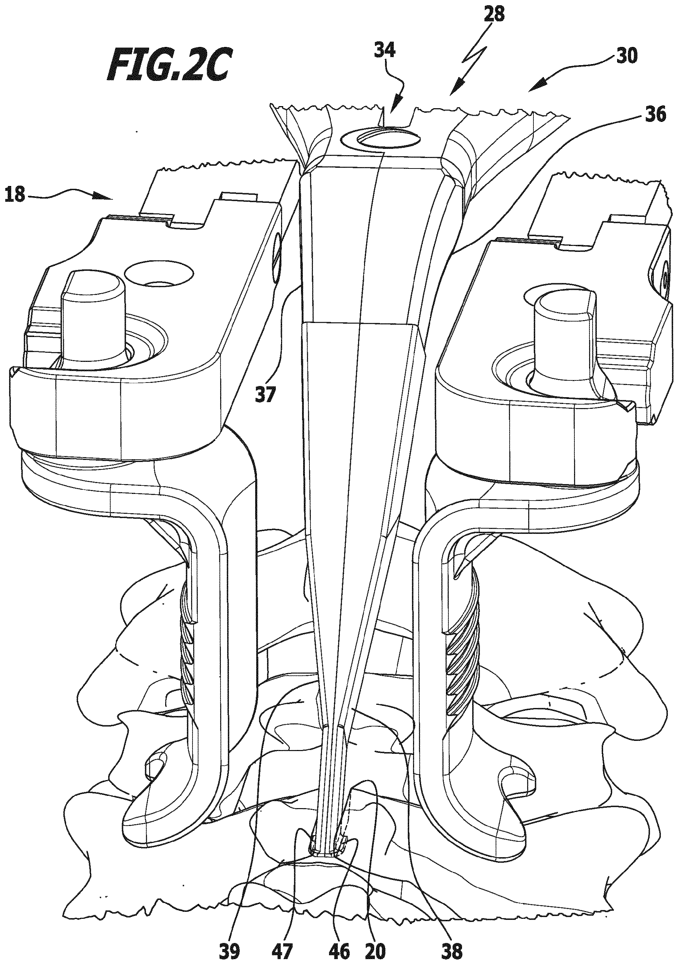

Zur elastisch/plastischen Aufweitung des Schnittspalts 20 wird ein erfindungsgemäßes Distraktionsinstrument 28 verwendet und ungefähr in Richtung der Sagittalebene in den Schnittspalt 20 eingeführt, wie dies in Figur 2C gezeigt ist.

Das Distraktionsinstrument 28 weist ein Griffteil 30 mit zwei an einem Hebelmechanismus 34 gegeneinander verschwenkbar gelagerten Griffbranchen 32, 33 auf, an dessen distalem Bereich relativ gegeneinander verschwenkbare Branchen 36, 37, an denen Werkzeugelemente 38, 39 gehalten, insbesondere angeformt sind.

Die Werkzeugelemente 38, 39 weisen einen sich in Schwenkrichtung zu ihrem distalen oder freien Ende oder Endbereich 40 hin konisch verjüngenden Querschnitt auf, der es erlaubt die Werkzeugelemente 38, 39 ohne größeren Kraftaufwand in einen zuvor gebildeten Schnittspalt einzuführen.

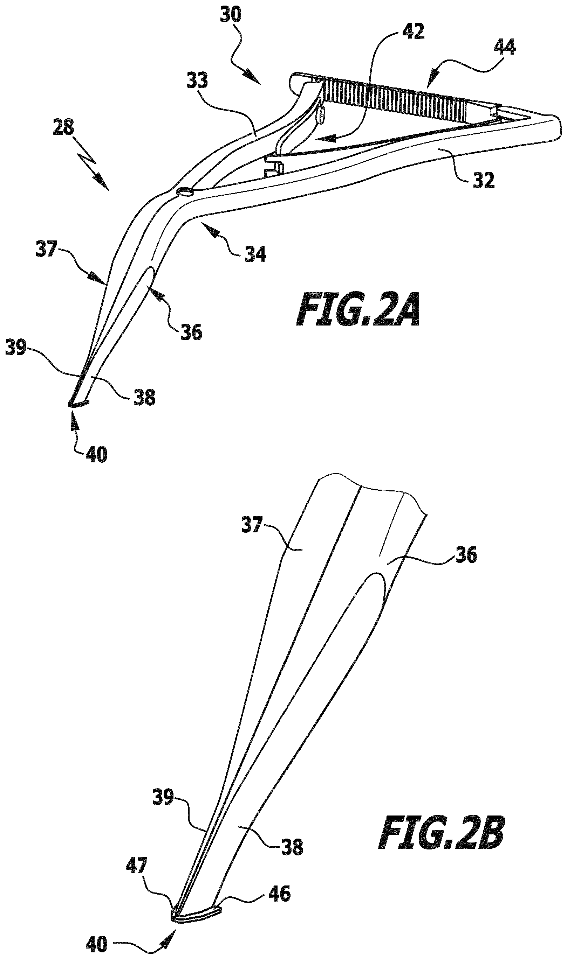

Die Griffelemente werden bevorzugt mittels einer Blattfederanordnung 42 gegeneinander vorgespannt in einer geschlossenen Ruhelage gehalten, bei der die beiden Werkzeugelemente 38, 39 aneinander anliegen, wie in den Figuren 2A und 2B gezeigt.

Das Griffteil 30 weist des Weiteren einen Rastmechanismus 44 auf, mit dem sich ein stufenweises Verstellen der Griffbranchen 32, 33 realisieren lässt, und davon abgeleitet ein stufenweises Aufweiten des Schnittspalts unter Ausnutzung der viskoelastischen Eigenschaften der Knochensubstanz des Wirbelbogens.

Alternativ oder in Kombination mit der Rastvorrichtung 44 kann eine Spaltbreitenanzeige realisiert sein und/oder eine Kraftmessvorrichtung, so dass die Werkzeugelement-seitig erreichte Spaltbreite am Griffteil 30 ablesbar ist bzw. die bei der Aufweitung aufgewendete Kraft dosiert eingesetzt werden kann. Die Rastvorrichtung 44 fixiert vorzugsweise eine einmal erreichte Aufweitposition des Distraktionsinstruments selbsttätig.

In Figur 2B sind die Werkzeugelemente 38, 39 und die distalen Enden der sie haltenden Branchen 36, 37 im Einzelnen gezeigt.

Bevorzugt weisen die Werkzeugelemente 38, 39 an ihrem Endbereich 40 seitlich abstehende Anlageflächen 46, 47 auf, die beim und nach dem Einführen der Werkzeugelemente 38, 39 in den Schnittspalt 20 auf der Spinalkanalseite der Wirbelbogenabschnitte anliegen, wie dies im Einzelnen in Figur 2C gezeigt ist.

Damit ist das Distraktionselement 28 an der Spinalkanal-seitigen Wand der Wirbelbogenabschnitte geführt und in einer Position gehalten, in der dann die Aufweitung des Schnittspalts sicher erfolgen kann.

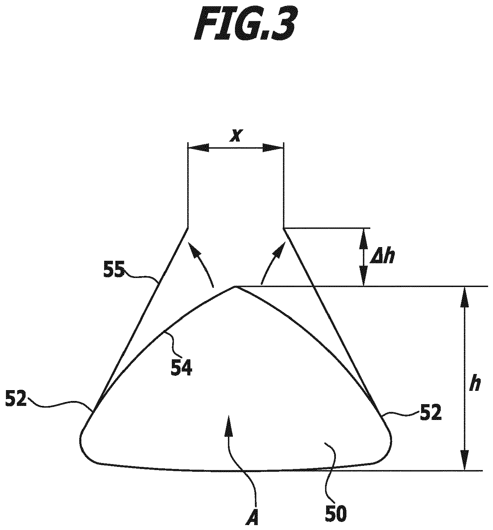

Die Ergebnisse bezüglich der Aufweitung des Wirbelkanaldurchmessers bzw. der Wirbelkanalfläche lassen sich an einem einfachen Modell anhand der Figur 3 erläutern.

Ausgangspunkt ist ein C6-Wirbel mit den in Figur 3 indizierten Parametern A = 150,65 mm² und einem Durchmesser h von 11,5 mm. Die Berechnungen für eine entsprechende Spaltbreite x basieren auf folgenden Annahmen:
- Die Gestalt des Spinalkanals im Wirbel kann durch ein gebogenes Dreieck 50 wie aus Figur 3 ersichtlich angenähert werden.
- Der Drehpunkt 52 der Wirbelbogenabschnitte liegt im Bereich der so genannten facet joints oder Füßchen.
- Die einzige elastisch/plastische Deformation der Abschnitte des Wirbelbogens 12 wird im Bereich der Lamina angenommen, wobei deren Bogenlänge der Einfachheit halber als konstant angenommen wurde und die Biegelinien 54, 55 als Kurven vereinfacht wurden.
- Der Wirbelkörper (nicht gezeigt) und die Anschlussstellen der Lamina an den Wirbelkörper (Füßchen) werden als starr angenommen.

In der Berechnung wurde die Öffnungsweite (Spaltbreite) x im Bereich von 6 bis 16 mm in 2 mm-Inkrementen erhöht. Die entsprechenden Werte für den Flächenzuwachs ΔA bzw. den Zuwachs an Durchmesser Δh sind in der folgenden Tabelle 1 aufgelistet.

Diese Werte zeigen, dass der in der Literatur (Wang, Xiang-Yang et al. in Spine Vol. 31, Nr. 34, 2006, Seiten 2863 bis 2870) empfohlene Wert für den Durchmesserzuwachs mit der elastisch/plastischen Verformung der Knochensubstanz erzielbar ist.

**Tabelle 1**

| **Spaltbreite** | **Flächenzuwachs** | **Durchmesserzuwachs** |
|---|---|---|
| x | ΔA | Δh |
| 6 mm | 50,67 mm² | 3,79 mm |
| 8 mm | 63,30 mm² | 4,22 mm |
| 10 mm | 75,36 mm² | 4,53 mm |
| 12 mm | 86,72 mm² | 4,72 mm |
| 14 mm | 107,66 mm² | 4,99 mm |
| 16 mm | 126,84 mm² | 5,16 mm |

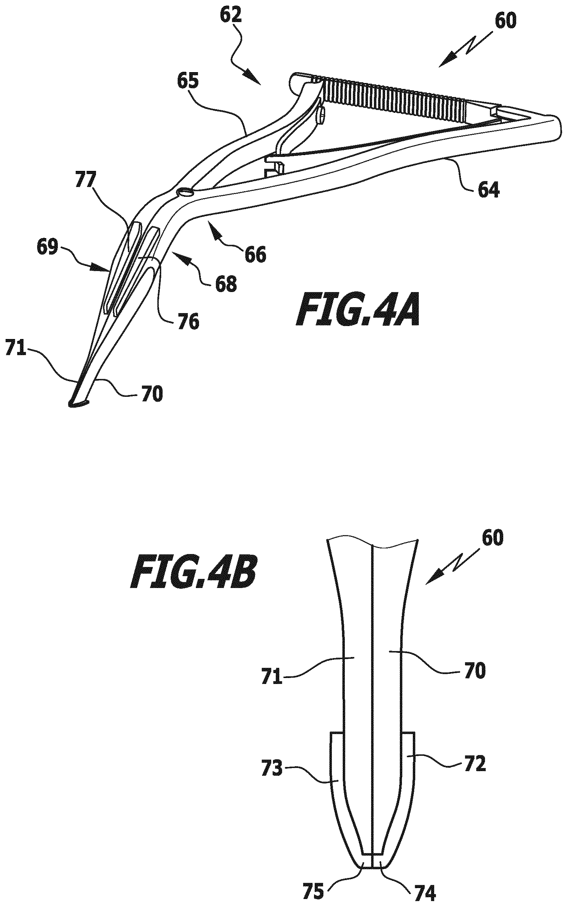

In Figur 4A ist eine alternative Ausführungsform eines erfindungsgemäßen Distraktionsinstruments 60 gezeigt mit einem Griffteil 62, dessen Griffteile 64, 65 über ein Schwenklager 66 miteinander verbunden sind. Distal zum Schwenklager 66 sind Branchen 68, 69 angeformt, an deren distalseitigem Ende Werkzeugelemente 70, 71 gehalten sind. Die freien Enden der Werkzeugelemente 70, 71 können wiederum so gestaltet sein wie bei der vorhergehenden Ausführungsform beschrieben, insbesondere mit Anlageelementen, die seitlich von den Werkzeugelementen 70, 71 abstehen, versehen sein.

In der Figur 4B sind die Werkzeugelemente 70, 71 nochmals im Einzelnen gezeigt sowie an diese seitlich angeformte Anlageflächen in Form von Flanschen 72, 73. Anders als bei der Ausführungsform des Distraktionsinstruments 28 ist beim Distraktionsinstrument 60 der Querschnitt der Werkzeugelemente nicht im Wesentlichen rechteckig, sondern keilförmig ausgebildet, so dass die Breite der geschlossenen Werkzeugelemente an ihrer vorderen, d.h. zunächst in den Schnittspalt einzuführenden, Seite geringer ist als an dem entgegengesetzt liegenden Ende.

Weiter weist das erfindungsgemäße Distraktionsinstrument 60 an seinen Werkzeugelementen 70, 71 Anschlagselemente 74, 75 auf, die nach vorn abstehen und direkt an die Anlageflächen 72, 73 angeformt sind.

Ferner weist das erfindungsgemäße Distraktionsinstrument 60 an der Oberseite der Branchen 68, 69 Führungselemente 76, 77 auf, die beispielsweise aus Kunststoff gefertigt werden können.

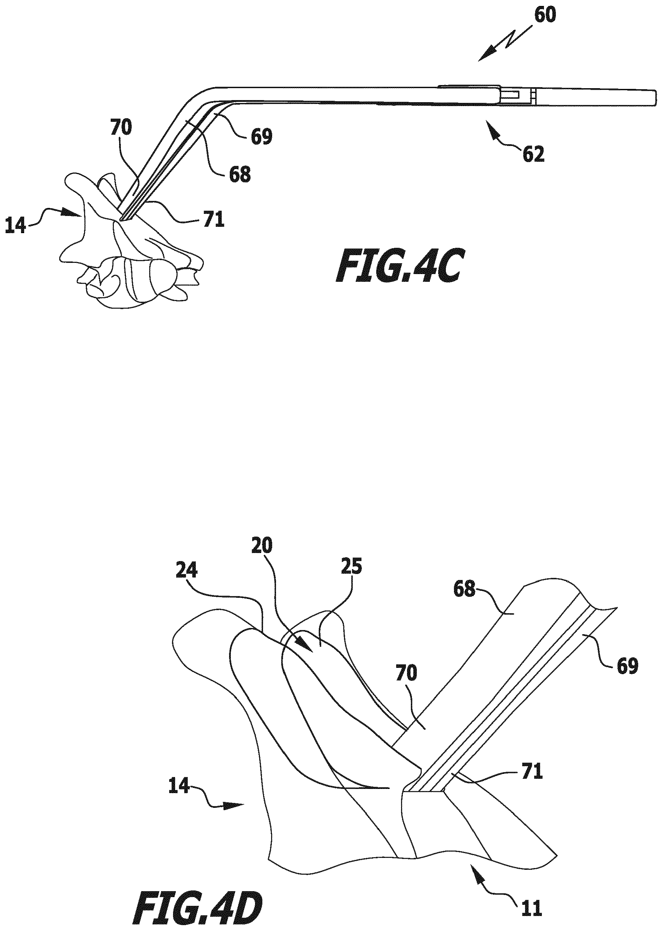

Das Einsetzen eines erfindungsgemäßen Distraktionsinstruments ist anhand des Distraktionsinstruments 60 in Figur 4C gezeigt. Figur 4C veranschaulicht die Einführung des Distraktionsinstruments 60 in sagittaler Richtung, wobei die Werkzeugelemente 70, 71 mit ihren Anlageflächen 72, 73 unter die Wirbelbögen greifen, d.h. Spinalkanal-seitig am Wirbelbogen des Wirbels 14 anliegen. Dies ist im Detail in Figur 4D nochmals veranschaulicht.

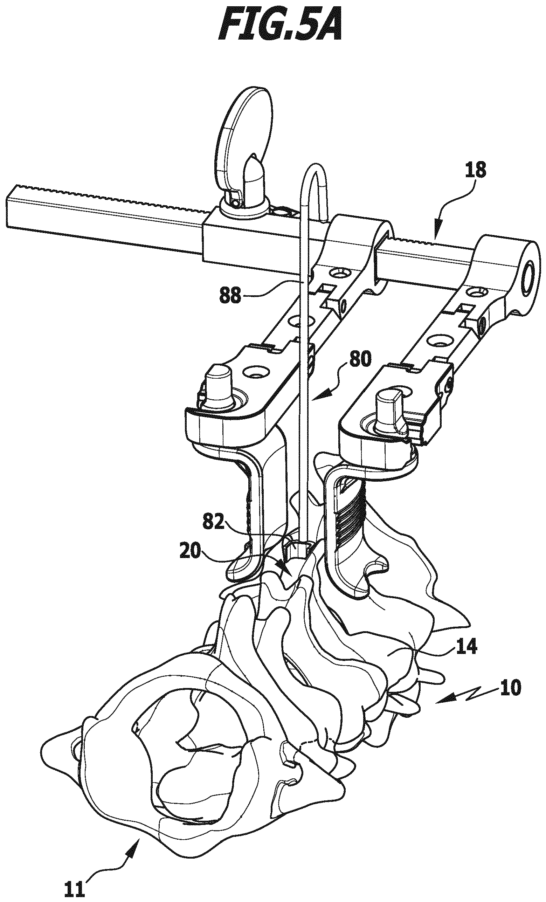

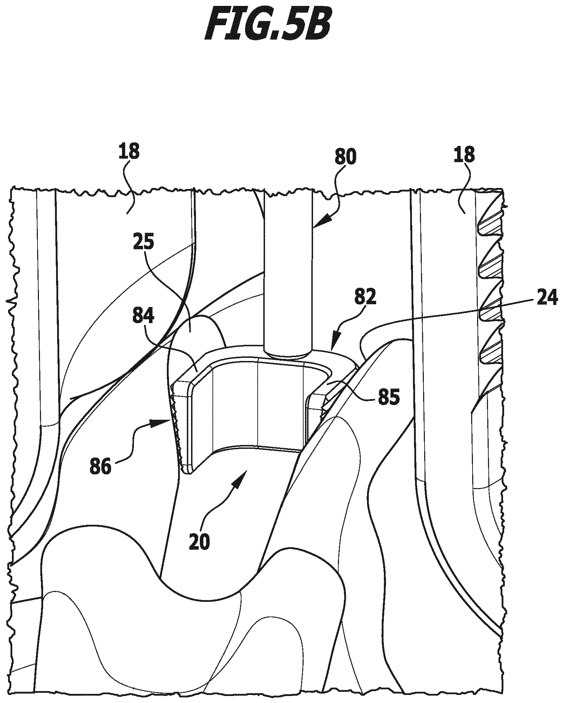

Ist der Schnittspalt 20 ausreichend aufgeweitet, kann er in diesem Zustand nach einer Variante der Erfindung durch einen zum erfindungsgemäßen Instrumentarium gehörenden Abstandhalter 80 stabilisiert werden, wie dies in den Figuren 5A und 5B gezeigt ist.

Der Abstandhalter 80 weist bevorzugt ein U-förmig gebogenes Abstandselement 82 auf, das die Schnittflächen 24, 25 des Schnittspalts 20 auf einem vorgegebenen Abstand hält. In diesem Zustand ist der Rückenmarkskanal im Bereich des Wirbels 14 frei zugänglich. Die beiden Schenkel 84, 85 des U-förmigen Abstandselements 82 sind vorzugsweise großflächig ausgebildet, so dass der Kontakt zwischen den Schnittflächen 24, 25 des Schnittspalts 20 und dem Abstandselement möglichst großflächig ist und bei den notwendigen Kräften zur elastisch/plastischen Aufweitung des Wirbelkanals eine möglichst kleine Flächenpressung resultiert. Diesem Ziel dient auch die bevorzugte Maßnahme, die außen liegenden Oberflächen des Abstandselements 82 in Keilform gegeneinander geneigt anzuordnen, entsprechend der Neigung der Schnittflächen 24, 25 des aufgeweiteten Schnittspalts 20.

Um das Abstandselement 82 möglichst sicher in seiner Position zwischen den Schnittflächen im Schnittspalt zu halten, weisen die zu den Schnittflächen 24, 25 weisenden Oberflächen mehrere parallele Rippen 86 auf, die einem unbeabsichtigten Herausgleiten des Abstandelements aus dem Schnittspalt in dorsaler Richtung entgegenwirken. Aufgrund der Parallelität der Rippen 86 lässt sich das Abstandselement 82, sobald das Implantat im Schnittspalt 20 eingesetzt ist, ohne größeren Kraftaufwand in sagittaler Richtung aus dem Schnittspalt 20 entfernen.

Eine zusätzliche Sicherung des Abstandselements in seiner in dem Schnittspalt eingesetzten Position kann durch einen vorzugsweise am bogenförmigen Teil des Abstandselements 82 angeformten schaftartigen Halter 88 erzielt werden, der bei der Verwendung eines Retraktors 18, wie z.B. in Figur 5A gezeigt, an diesem bedarfsweise festgelegt werden kann.

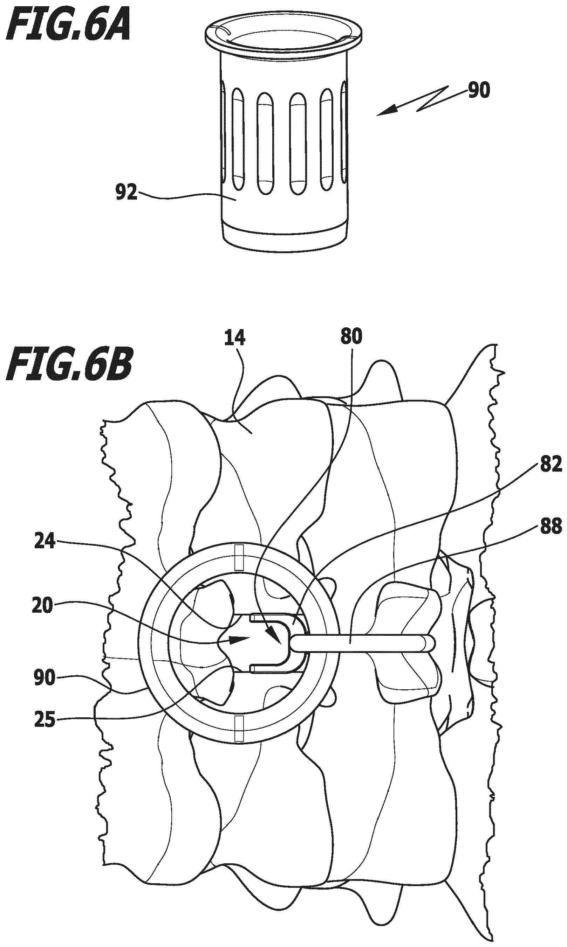

Die Figur 6A zeigt einen alternativen Retraktor in Form einer Retraktorhülse 90, die einfach in den Gewebeschnitt oberhalb des zu operierenden Wirbelkörpers 14 eingesetzt wird und dann das Gewebe rings um die Operationsstelle mit ihrem zylindrischen Körper 92 zurückhält.

In Figur 6B ist der Retraktor 90 in seiner typischen Verwendungsposition gezeigt (allerdings ohne das umgebende Gewebe).

Der Retraktor 90 mit seinem zylindrischen Körper oder der Hülse 92 ist in einigen Fällen völlig ausreichend, um den Operationsbereich wie beschrieben freizuhalten. Er hat außerdem den Vorteil, dass er sich nicht in das Umfeld des Operationsbereichs erstreckt und dieses im Wesentlichen frei zugänglich lässt.

Figur 6B zeigt den Abstandhalter 80 mit dem im Schnittspalt 20 positionierten Abstandselement 82 bei Verwendung des Retraktors 90. Die Schnittflächen 24, 25 werden auf einem vorgegebenen Abstand gehalten, während das U-förmige Abstandselement freie Sicht und Zugang zum Spinalkanal gewährleistet.

Die Figur 6B zeigt in der Draufsicht den durch den elastisch aufgeweiteten Schnittspalt 20 geschaffenen Zugang zu dem Rückenmarkskanal 11, der sich in gleicher Weise natürlich auch bei der Verwendung eines Retraktors anderen Typs, wie z.B. des Retraktors 18, ergibt.

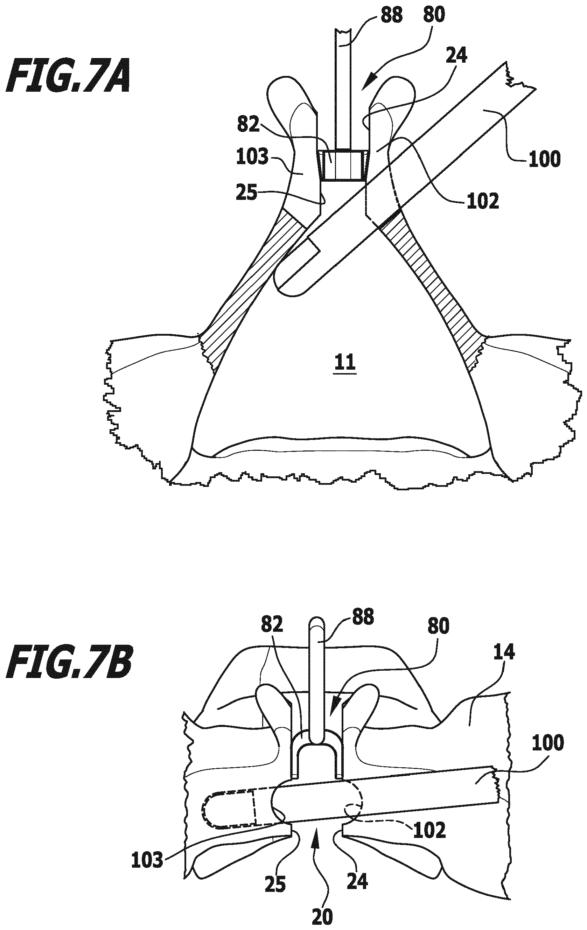

Die Figuren 7A und 7B veranschaulichen schematisch in Seitenansicht bzw. Draufsicht die Möglichkeit des Zugangs zum Rückenmarkskanal 11 am Beispiel eines so genannten Shavers 100. Um bei gleicher Spaltbreite einen größeren Aktionsradius für den Einsatz chirurgischer Instrumente, beispielsweise eines Shavers oder einer Stanze, zu schaffen und zur Erlangung von weiteren, im Folgenden noch zu diskutierenden Vorteilen, werden in den Schnittflächen 24, 25 des Schnittspalts 20 bevorzugt Nuten 102, 103 ausgebildet, die sich weiter bevorzugt über die gesamte Höhe der Schnittflächen 24, 25 erstrecken.

Beispielsweise kann mit dem Shaver 100 wie in den Figuren 7A und 7B gezeigt die Lamina auf der Spinalkanalseite des Wirbelbogens bearbeitet werden, um so Raum für eine weitere Dekompression des Rückenmarks zu schaffen.

Dies ermöglicht ein Abtragen von Knochensubstanz auf der Spinalkanalseite der Lamina, um die Elastizität derselben zu erhöhen und eine weitere Aufweitung zu ermöglichen. In schwierigen Fällen kann auch erwogen werden, eine Kerbe auf Seiten des Spinalkanals zu schaffen, so dass sich die Wirbelbogenabschnitte ähnlich wie bei der konventionellen Double-Door-Technik um eine Art Scharnier aufbiegen lassen. Im Gegensatz zur konventionellen Technik wird dazu allerdings keine Ablösung der Muskulatur von der Wirbelsäule notwendig, so dass nach wie vor ein schonenderer Eingriff vorliegt.

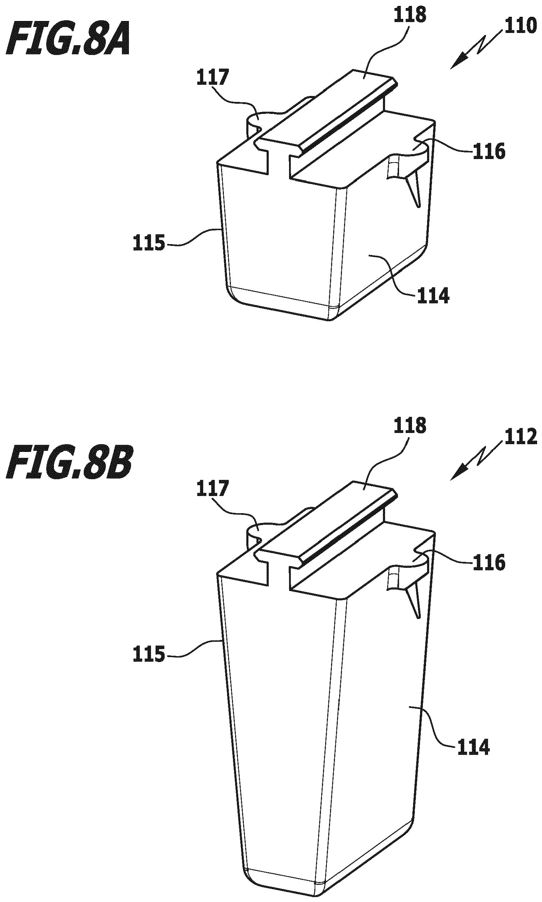

Ist der aufgeweitete Schnittspalt 20 bereit für das Einsetzen eines Implantats, so wird vorzugweise ein Implantatkörper 110 oder 112, wie beispielhaft in den Figuren 8A und 8B dargestellt, in den Schnittspalt 20 eingesetzt.

Die beiden Implantatkörper 110, 112 sind beide massiv ausgebildet und vorzugsweise aus einem Implantat-tauglichen Kunststoff, insbesondere PEEK, hergestellt. Die beiden Implantatkörper 110, 112 weisen gegeneinander geneigte Kontaktflächen 114, 115 auf, die mit den Schnittflächen des Schnittspalts im eingesetzten Zustand des Implantats in möglichst großflächigem Kontakt stehen. Während die Implantatkörper 110, 112 in Frontansicht keilförmig gestaltet sind, können sie in der Seitenansicht durchaus im Wesentlichen rechteckig ausgebildet sein.

Der Implantat-taugliche Kunststoff (z.B. PEEK) ist bevorzugt zur Förderung des Anwachsens der Knochensubstanz am Implantat beschichtet, beispielsweise mit der Plasmapore-Technik oder mittels einer Hydroxyapathitschicht.

An ihrem in den Figuren 8A und 8B oberen Ende, das im eingesetzten Zustand des Implantats dorsal liegt, weisen die Implantatkörper 110, 112 seitlich abstehende Vorsprünge 116, 117 auf, die mehrere Funktionen übernehmen können:
Zum Einen bewirken die Vorsprünge 116, 117, dass das Implantat nur bis zum Anschlag der Vorsprünge 116, 117 an der Knochensubstanz in den Schnittspalt eingeführt werden kann und auch in der nachoperativen Phase eine Verlagerung des Implantats in Richtung des Spinalkanals unterbunden wird.

Des Weiteren können die Vorsprünge 116, 117 im Formschluss mit einem Instrument oder mit entsprechenden Rücksprüngen in der Knochensubstanz eine Führung des Implantatkörpers beim Einsetzen des Implantats in den Schnittspalt bewirken und so eine präzise Platzierung unterstützen.

Schließlich vergrößern die Vorsprünge den dorsalen Bereich des Implantatkörpers und erleichtern somit die Aufnahme von Halteelementen wie z.B. Schrauben, Spikes etc., die der Fixierung des Implantats im aufgeweiteten Schnittspalt dienen.

Dorsal abstehend ist an den Implantatkörpern 110, 112 eine beidseits hinterschnittene Leiste 118 als Griffelement angeformt, die der besseren Handhabung beim Einsetzen und dem korrekten Positionieren des Implantats dient.

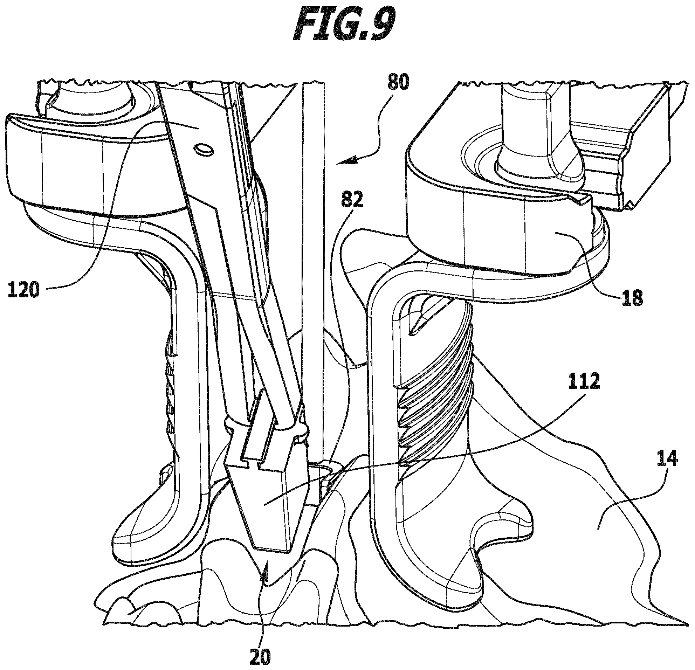

Figur 9 zeigt den Vorgang des Einsetzens des Implantatkörpers 112 in den Schnittspalt 20, der von einem Abstandhalter 80 in aufgeweitetem Zustand gehalten wird. Um für das Einsetzen des Implantats genügend Raum zu schaffen, kann dazu der Abstandhalter 80 in sagittaler Richtung ein Stück weit aus dem Schnittspalt heraus bewegt werden, so dass nur noch kleinere Flächenanteile der Schenkel des Abstandselements 82 im Kontakt mit den Schnittflächen 24, 25 des Schnittspalts 20 stehen. Das Implantat wird dann, wie in Figur 9 gezeigt, mit einer Zange 120 in leichter Neigung gegen die Schnittspaltachse in den Schnittspalt 20 eingeführt.

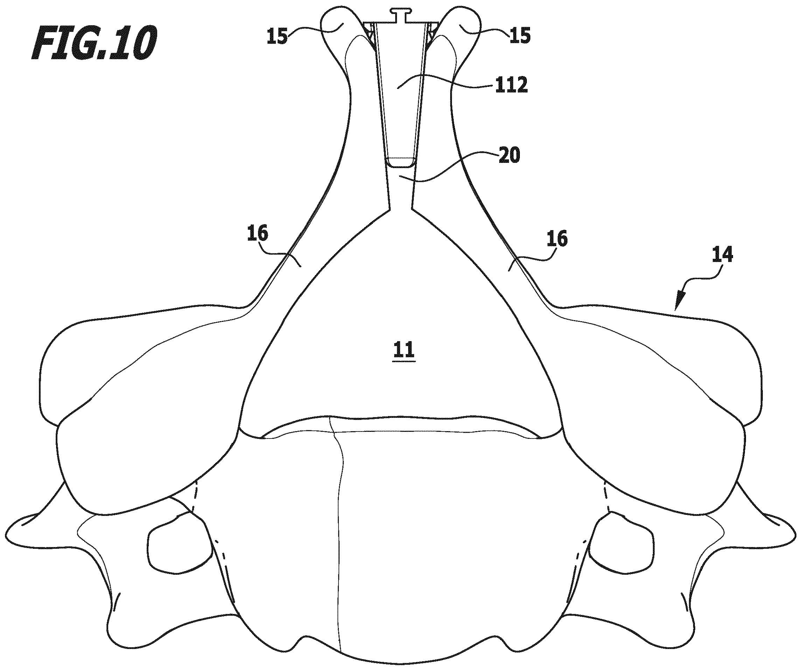

Nach einer vollständigen Entnahme des Abstandhalters 80 wird der Implantatkörper 112 in seine endgültige Position gebracht, wie sie in Figur 10 dargestellt ist. Die Höhe des Implantatkörpers 112 ist in diesem Beispiel so gewählt, dass er im eingesetzten Zustand mit seinem ventral liegenden Ende nicht ganz an den Spinalkanal heranreicht, so dass dort ein zusätzliches Volumen für die Dekompression des Rückenmarks verbleibt.

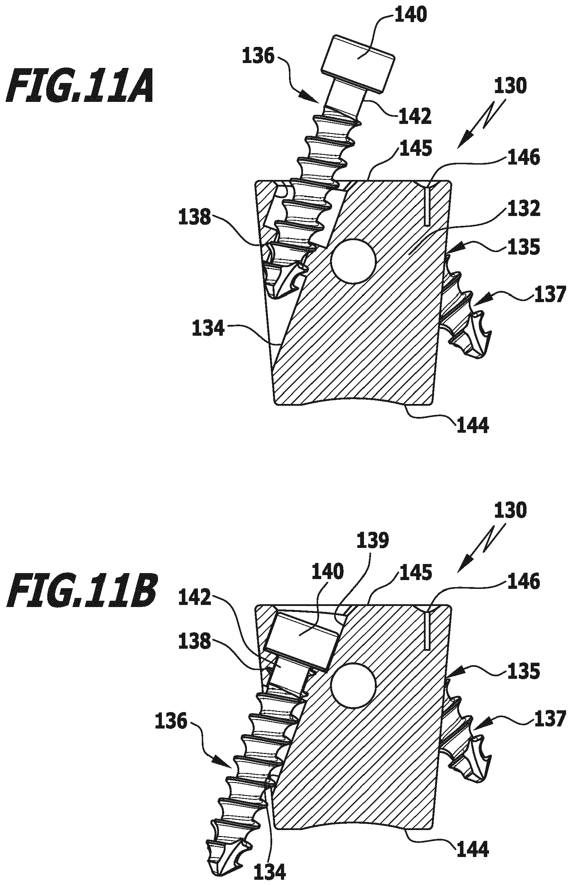

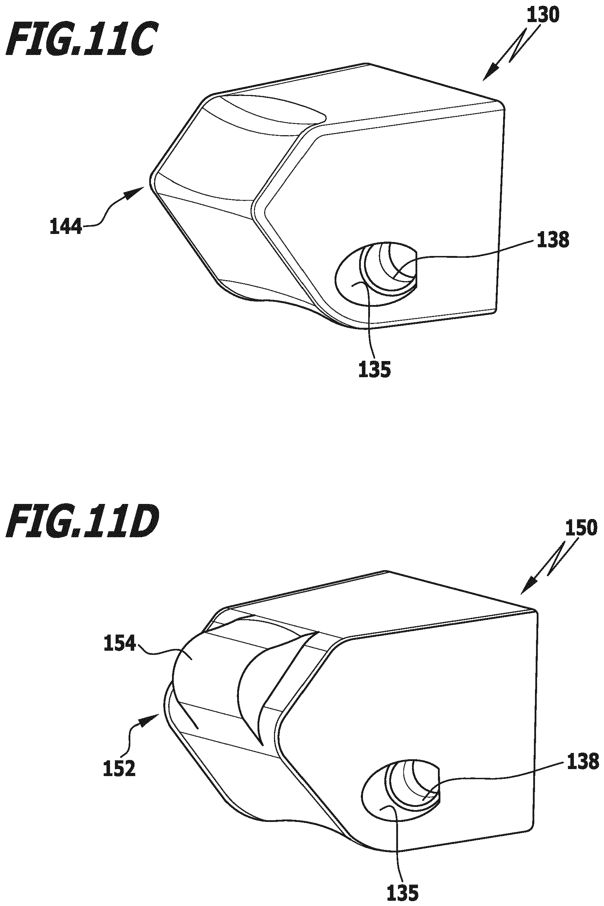

Die Abbildungen A und B der Figur 11 zeigen eine Schnittansicht durch ein Implantat 130 mit zwei gegenüber den Kontaktflächen des Implantatkörpers 132 spitzwinklig verlaufende Durchgangsöffnungen 134, 135. Die beiden Durchgangsöffnungen 134, 135 sind gestaffelt angeordnet, so dass in den Schnittdarstellungen A und B der Figur 11 nur der in der Zeichnung vorn liegende Durchgangskanal 134 vollständig sichtbar ist. In beiden Durchgangsöffnungen 134, 135 sind Schraubbolzen 136, 137 angeordnet, von denen der Schraubbolzen 137 bereits vollständig in die Durchgangsöffnung 135 eingeschraubt ist und mit seinem spitzen Ende über die Kontaktfläche des Implantats 130 hinaus in die Knochensubstanz eines Wirbelbogens (hier nicht dargestellt) eindringt.

Der Schraubbolzen 136 ist in der Darstellung A der Figur 11 noch in seiner Ausgangsstellung gezeigt, in der er von einem kurzen Gewindeabschnitt 138, der einen Teil der Durchgangsöffnung 134 bildet, formschlüssig gehalten wird.

Nachdem der Implantatkörper 132 in einen Schnittspalt eines Wirbelbogens eingesetzt ist, werden die beiden Schraubbolzen 136, 137 eingeschraubt und so das Implantat 130 an der umliegenden Knochensubstanz fixiert. Der Gewindeabschnitt 138 führt dabei die Schraubbolzen zusammen mit den restlichen Teilen der Durchgangsöffnungen 134, 135, so dass sie in einer vorgegebenen Orientierung in die umgebende Knochensubstanz eindringen können.

Die Durchgangsöffnungen 134, 135 weisen an ihren dorsal liegenden Öffnungen an der Oberfläche des Implantats 130 einen größeren Durchmesser auf, so dass die Schraubbolzen 136, 137 im eingeschraubten Zustand mit ihrem Schraubenkopf 140 ganz innerhalb des Körpers des Implantats 130 aufgenommen werden können. An diesen Bereich 139 mit erweitertem Durchmesser der Durchgangsöffnung 134, 135 schließt sich der zuvor schon erwähnte Abschnitt mit einem Innengewinde 138 an.

Die Schraubbolzen 136, 137 werden vorzugsweise so gestaltet, dass an ihrem dem Schraubenkopf 140 benachbarten Bereich ein so genannter Freilauf 142 vorgesehen ist, der lang genug ist, um das Innengewinde 138 der Durchgangsöffnung 134, 135 zu durchsetzen, so dass bei vollständig eingeschraubten Schraubbolzen 136, 137 (Figur 11B) das Gewinde der Schraubbolzen außer Eingriff mit dem Innengewinde 138 gerät und dadurch der Implantatkörper 132 durch Anziehen der Schraubbolzen 136, 137 zu einer engen Anlage mit seinen Kontaktflächen an die Schnittflächen der Knochensubstanz gebracht werden kann.

Die Länge der Schraubbolzen 136, 137 ist so bemessen, dass sie auch nach vollständigem Einschrauben in den Implantatkörper 132 nicht so weit aus dem Implantatkörper herausstehen, dass sie in den Spinalkanal eindringen könnten. Vorzugsweise ist deshalb die Spitze der Schraubbolzen 136, 137 in vollständig eingeschraubtem Zustand hinter der ventralen Vorderkante 144 des Implantats 130 zurückbleibend positioniert.

Die Figur 11C zeigt das Implantat 130 der Figur 11A in perspektivischer Darstellung mit seinem ventralen bzw. distalen Endbereich 144 (Vorderkante), der mit einer Einbuchtung versehen ist, so dass zusätzliches Volumen benachbart zum Spinalkanal geschaffen wird.

In der Alternative der Figur 11D weist das Implantat 150 eine im Wesentlichen ähnliche Struktur zu dem Implantat 130 auf, mit dem Unterschied, dass am ventralen Endbereich 152 eine Nase 154 angeformt ist, welche beim Einführen des Implantats in den Schnittspalt des Wirbelbogens zwischen die Branchen einer Distraktionszange eingreifen kann, so dass das Implantat 150 geführt, insbesondere zentriert in den Schnittspalt des Wirbelbogens eingeführt werden kann.

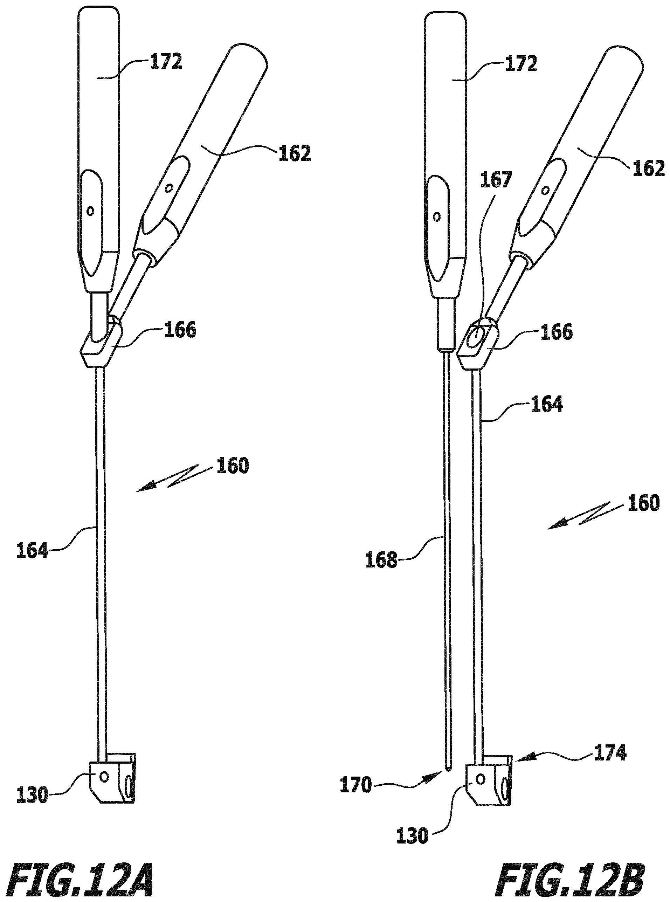

Um die Implantate besser handhaben zu können, werden diese bevorzugt an einem Halte- oder Einführinstrument befestigt, der Teil des erfindungsgemäßen Instrumentariums ist. Bevorzugte Implantate weisen zur Verbindung mit dem Halte- bzw. Einführinstrument entsprechende Merkmale auf, beispielsweise eine Bohrung in der dorsal gelegenen Endfläche 145, wie dies in der Figur 11A und 11B beispielhaft als Bohrung 146 mit Innengewinde dargestellt ist. In dieser lässt sich ein Halte- bzw. Einführinstrument parallel zur Längsrichtung des Implantats fixieren, die im Zusammenhang mit den Figuren 12 bis 14 noch näher erläutert werden.

An seinem ventralen Ende 144 weist der Implantatkörper 130 eine Einbuchtung 148 auf, die sich um die ventral vorstehende Kante erstreckt und eine weitere Vergrößerung des auf Seiten des Spinalkanals verfügbaren Raums und somit eine weitere Dekompression des Rückenmarks erlaubt. Mit dieser Maßnahme seitens des Implantatkörpers 130 wird ein zusätzlicher Raumgewinn für den Spinalkanal realisiert, der ansonsten nur durch ein wesentlich stärkeres Aufspreizen des Wirbelbogens erzielt werden könnte.

Die Figuren 12A und B zeigen ein Halte- bzw. Einführinstrument 160 mit einem Handgriff 162 und einem von dessen distalem Ende abgewinkelt abstehenden Schaft 164. Der Schaft 164 ist als rohrförmiges Teil ausgebildet und ist mit seinem proximalen Ende mit dem Handgriff 162 über ein Verbindungsstück 166 verbunden, welches fluchtend zum Schaft 164 auf der von diesem abgewandten Seite eine Durchgangsbohrung 167 aufweist, über die der Hohlraum in den Schaft 164 zugänglich ist.

Das Halte- bzw. Einführinstrument 160 weist des Weiteren einen Gewindeschaft 168 auf, der in den hohlzylindrischen Schaft 164 über die Durchgangsöffnung 167 eingeführt werden kann und der bis zum distalen Ende des Schaftes 164 reicht.

An seinem distalen Ende 170 weist der Gewindeschaft 168 einen Gewindeabschnitt auf, während an seinem proximalen Ende ein Handgriff 172 koaxial befestigt ist. Am distalen Ende des Schaftes 164 ist schließlich ein Schlitten 174 montiert, der eine Aufnahme für ein Implantat 130 bildet.

Sobald das Implantat 130 auf dem Schlitten 174 positioniert ist, kann der Gewindeschaft 168 in die Durchgangsöffnung 167 bzw. den Schaft 164 eingesetzt werden und dessen Gewindeabschnitt am distalen Ende 170 in die Bohrung 146 des Implantats 136 eingeschraubt werden.

In diesem Zustand stützt sich der Handgriff 172 an dem Verbindungsstück 166 ab und fixiert so das Implantat 130 an dem Schlitten 174.

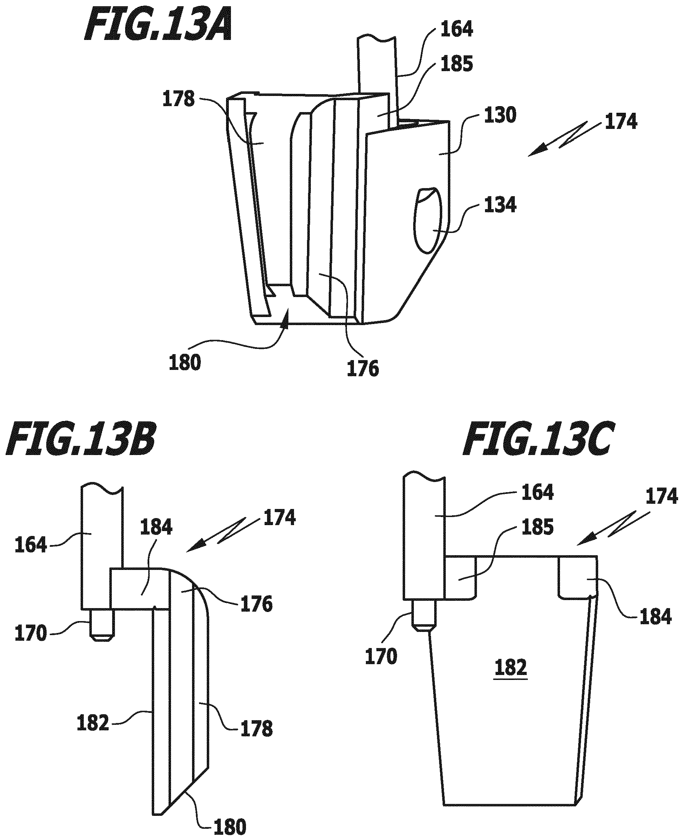

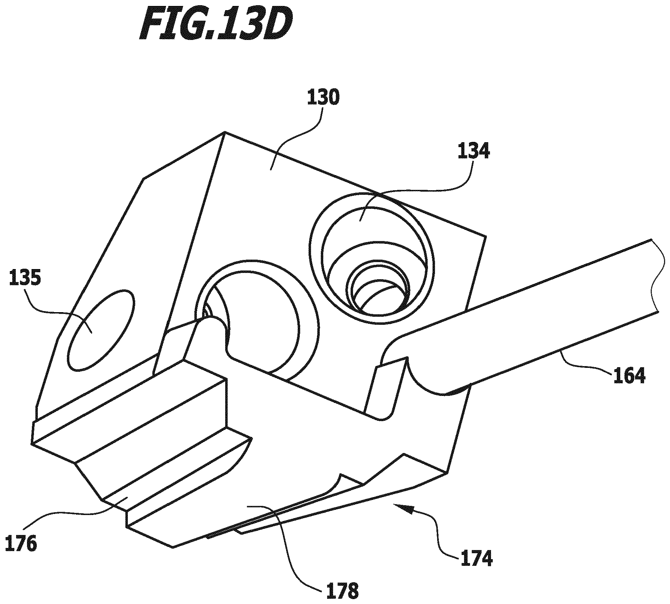

Einzelheiten des Schlittens 174 sind in den Figuren 13A bis 13D dargestellt. In den Figuren 13A und 13D ist am Schlitten 174 das Implantat 130 montiert, in den Figuren 13B und 13C ist der Schlitten 174 als solcher dargestellt. Auf der in Figur 13A vorn liegend dargestellten Rückseite des Schlittens 174 ist ein Zentrierungsvorsprung 176 vorgesehen, der zum Führen des Schlittens zwischen den Branchen eines erfindungsgemäßen Distraktionsinstrumentes vorgesehen ist. Vorzugsweise weist der Vorsprung 176 mittig und in Längsrichtung noch einen vorspringenden Absatz 178 auf, der der Vorzentrierung des Schlittens zwischen den Branchen des Distraktionsinstruments dient.

An seinem distalen Ende weist der Schlitten 174 eine Einführschräge 180 auf, die das Einführen des Schlittens 174 mit dem Implantat 130 in den aufgeweiteten Schnittspalt 120 dient bzw. dieses erleichtert. Die Einführschräge 180 ist in einem Winkel von ca. 45 ° gegenüber einer Anlagefläche 182 für das Implantat 130 geneigt. An seinem proximalen Ende weist der Schlitten 174 zwei über die Anlagefläche 182 vorspringende Stopper 184, 185 auf, die an der dorsalen Endfläche 145 des Implantatkörpers 130 anliegen und so dessen Position auf der Anlagefläche 182 definieren.

Der Stopper 185 ist dann mit dem Schaft 164 des Halte- bzw. Einführinstruments 160 fest verbunden. In den Figuren 13B und 13C ist am unteren Ende des Schaftes 164 überstehend der Gewindeabschnitt des distalen Endes 170 des Gewindeschaftes 168 des Einführ- bzw. Halteinstruments 160 zu sehen.

Figur 13D zeigt das auf dem Schlitten 174 montierte Implantat 130 ungefähr aus der Sicht des Operateurs.

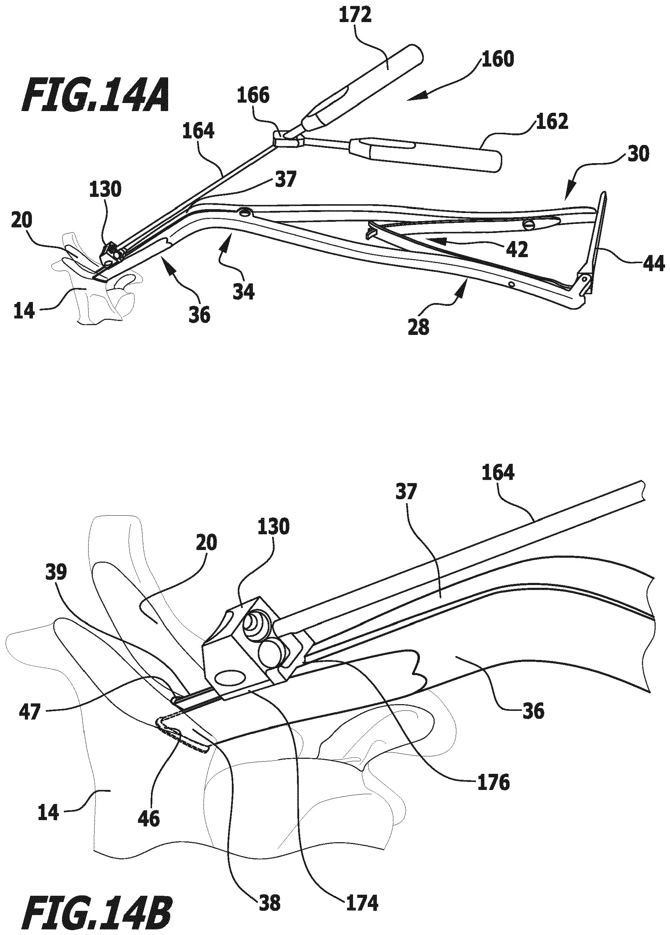

Die Figuren 14A bis C zeigen den Einsatz des erfindungsgemäßen Halte- und Einführinstruments 160 mit montiertem Implantat 130 im Zusammenhang mit einem im Schnittspalt 20 eines Wirbels 14 platzierten Distraktionsinstrument 28. Das Implantat 130 auf dem Schlitten 174 gleitet dabei entlang den Branchen 36, 37 und wird dabei durch den Vorsprung 176 zwischen den Branchen 36, 37 zentriert.

Mit diesem Halte- bzw. Einführinstrument 160 lässt sich das Implantat 130 einfach handhaben. Vorzugsweise wird dieses Halte- und Einführinstrument 160 zusammen mit einer Distraktionszange bzw. einem Distraktionsinstrument wie in der Figur 4A gezeigt verwendet, wobei dann das Implantat 130 selbst zwischen den dort vorgesehenen Führungselementen an der Oberseite der Branchen 68, 69 geführt wird.

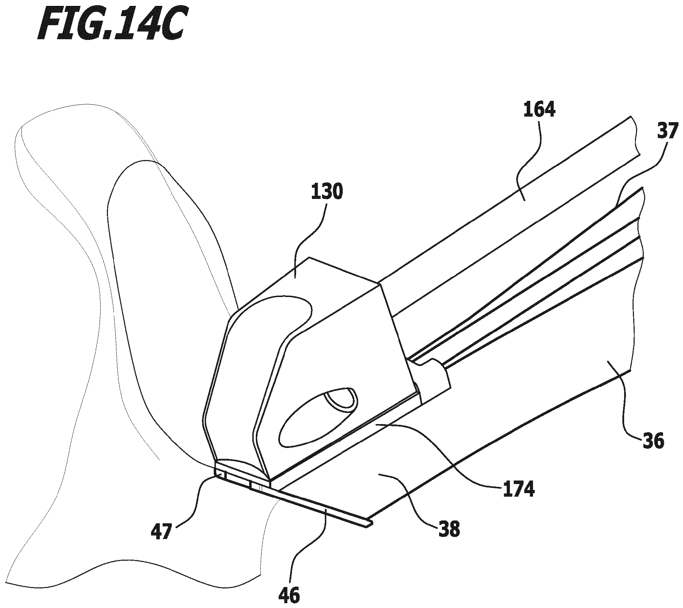

In der in Figur 14C dargestellten Situation, bei der zur Verdeutlichung der Endlage des eingesetzten Implantats 130 der eine Teil des Wirbelbogens, der vorn liegend wäre, weggelassen ist, zeigt das Implantat 130 bzw. der Schlitten 174 an den Anlageflächen 46, 47 der Werkzeugelemente 38, 39 positioniert. Erst in diesem Zustand, d.h. wenn das Implantat in seiner Endstellung im Schnittspalt angeordnet ist, wird das Halte- bzw. Einführinstrument 160 gelöst, indem der Gewindeschaft 168 mit seinem Ende 170 aus der Bohrung 146 des Implantats 130 heraus gedreht wird, so dass nachfolgend der Schlitten 174 mit dem Halte- bzw. Einführinstrument 160 aus dem Operationsfeld zurückgezogen werden kann.

Danach kann auch das Distraktionsinstrument 28 aus dem Schnittspalt 20 entfernt werden, wobei dieses wieder in Sagittalrichtung entfernt wird.

Falls das Implantat 130 noch nicht in der korrekten Stellung im Schnittspalt 20 angeordnet ist, wenn der Schlitten 174 die Anlageflächen 146 erreicht, wird dieses noch um den Anlagepunkt herum in die richtige Position gedreht.

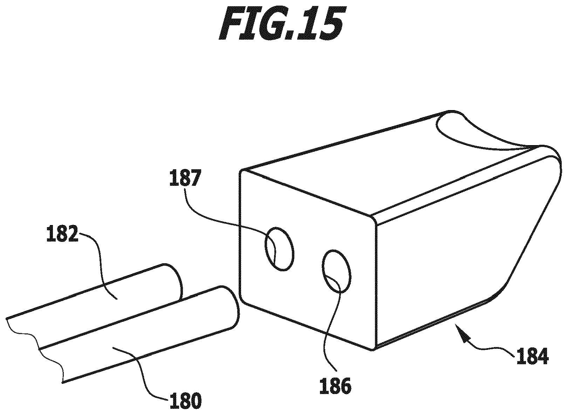

Figur 15 zeigt schematisch eine weitere Möglichkeit eines Halte- bzw. Einführinstruments mit zwei zylindrischen Branchen 180, 182 zusammen mit einem Implantatkörper 184, der von der dorsalen Seite her zwei Bohrungen 186, 187 aufweist, in denen die Branchen 180, 182 aufgenommen werden.

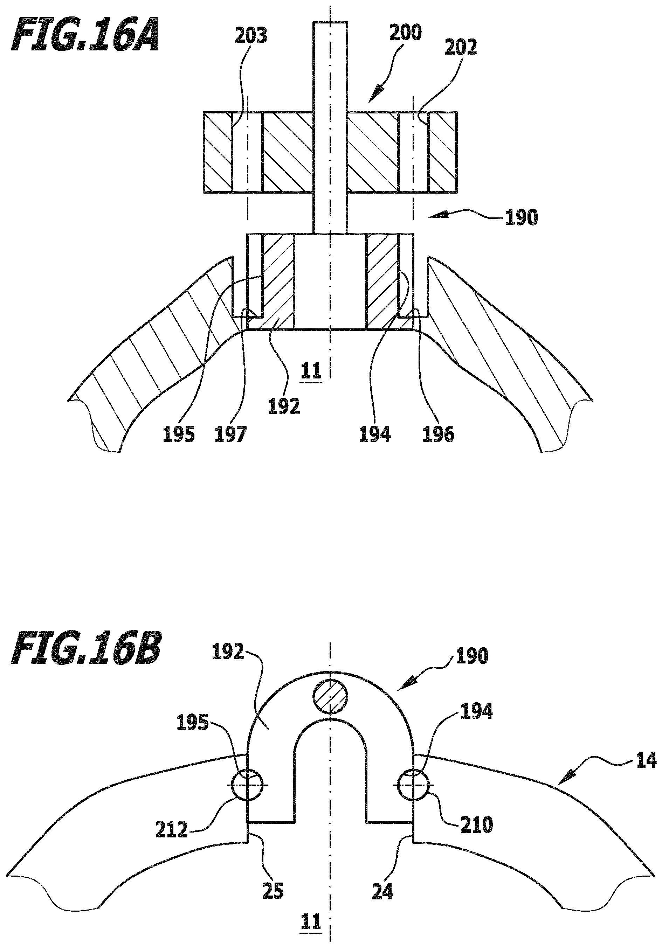

Die Figuren 16A und 16B zeigen einen alternativen Abstandhalter 190, der ähnlich wie der Abstandhalter 80 ein U-förmiges Abstandselement 192 aufweist.

An den außen liegenden Oberflächen des U-förmigen Abstandselements 192 sind halbzylindrische Ausnehmungen 194, 195 vorgesehen, die über einen Großteil der Höhe des Abstandselements 192 reichen, jedoch nach unten hin (zum Spinalkanal) über einen Anschlag 196, 197 geschlossen sind. Diese halbzylindrischen Ausnehmungen 194, 195 bieten zusammen mit einer Bohrlehre 200, die zu den halbzylindrischen Ausnehmungen 194, 195 fluchtende Durchgangsbohrungen 202, 203 aufweist, eine exakte Führung für ein Bohrwerkzeug (nicht dargestellt), mit dem in die Schnittflächen des Schnittspalts halbzylindrische Ausnehmungen in die Knochensubstanz eingebracht werden können. In diese Bohrungen 210, 212 lassen sich die Arbeitsenden 220, 222 oder Werkzeugelemente eines Distraktionsinstruments einsetzen, das den Schnittspalt dann zunächst in der aufgeweiteten Stellung hält. Nachfolgend kann ein entsprechend ausgebildetes Implantat 224 eingesetzt werden, das vorzugsweise ebenfalls halbkreisförmige Nuten 226, 227 an den Kontaktflächen aufweist. Über diese halbzylindrischen Nuten 226, 227 entlang den Arbeitsenden 220, 222 bzw. Werkzeugelementen eines Distraktionsinstruments geführt werden, bis es im Schnittspalt eingesetzt ist.

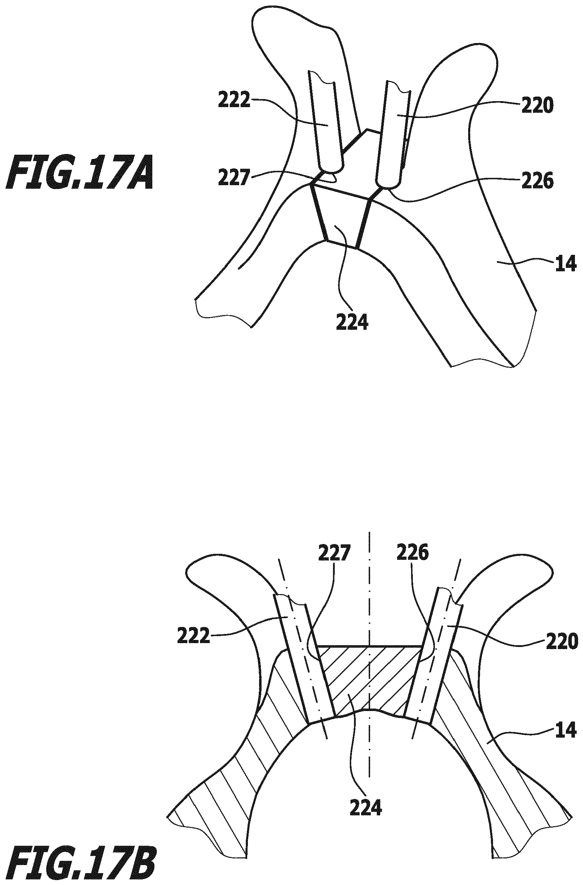

Eine solche Situation ist in den Figuren 17A und 17B gezeigt.

Nachfolgend kann das Distraktionsinstrument mit seinen zylindrischen Arbeitsenden 220, 222 abgezogen werden und die verbleibenden Bohrungen 210, 214 dienen dann dem Einsetzen von Schrauben oder Dübeln (nicht gezeigt) zur Fixierung des Implantats in dem Schnittspalt.

## Patentansprüche

1. Chirurgisches Instrumentarium zum Erweitern eines Wirbelkanals eines Wirbels im Rahmen einer Laminoplastie, umfassend ein Distraktionsinstrument (28;60) sowie ein Halteinstrument (160) für ein einzusetzendes Implantat, wobei in der Laminoplastie der Wirbelbogen eines Wirbels unter Bildung eines Schnittspalts durchtrennt und der Schnittspalt zum Einsetzen des an dem Halteinstrument (160) gehaltenen, insbesondere keilförmigen Implantats mit dem Distraktionsinstrument (28;60) aufgeweitet wird, wobei das Distraktionsinstrument proximal ein Griffteil (30;62) und distal zwei relativ zueinander bewegbare Branchen (68,69) mit daran gehaltenen Werkzeugelementen (38;39) aufweist sowie einen zwischen dem Griffteil (30;62) und den Branchen (68,69) angeordneten Hebelmechanismus, wobei die Branchen (68,69) Führungselemente (76,77) umfassen, welche ein geführtes Einsetzen des Implantats in den Schnittspalt ermöglichen, wobei die Werkzeugelemente (38,39;70,71) mit dem Hebelmechanismus relativ zueinander aus einer geschlossenen Ruhestellung in eine offene Spreizstellung bewegbar gehalten sind, und wobei die Werkzeugelemente (38,39;70,71) jeweils eine Biegefestigkeit aufweisen, die einer Distraktionskraft von ca. 300 N oder mehr entspricht, ohne dass eine plastische Verformung an den Werkzeugelementen (38,39;70,71) auftritt.

2. Instrumentarium nach Anspruch 1, **dadurch gekennzeichnet, dass** die Werkzeugelemente (38,39;70,71) an ihrem distalen Ende (40) eine Anlagefläche (46,47;72,73) zur Positionierung des Instruments (28;60) im Spalt (20) aufweisen.

3. Instrumentarium nach Anspruch 1 oder 2, **dadurch gekennzeichnet, dass** die Werkzeugelemente (70,71) an ihrem distalen Ende ein Anschlagselement (74,75) zur Positionierung eines Implantats im Schnittspalt (20) aufweisen.

4. Instrumentarium nach einem der Ansprüche 1 bis 3, **dadurch gekennzeichnet, dass** die Werkzeugelemente (38,39;70,71) einen in den Schnittspalt einführbaren Endbereich aufweisen, dessen Querschnitt keilförmig ausgebildet ist.

5. Instrumentarium nach einem der Ansprüche 1 bis 4, **dadurch gekennzeichnet, dass** die Werkzeugelemente (38,39;70,71) zu ihrem distalen Ende hin konisch verjüngt ausgebildet sind.

6. Instrumentarium nach einem der Ansprüche 1 bis 5, **dadurch gekennzeichnet, dass** die Längsachsen der die Werkzeugelemente (38,39;70,71) haltenden Branchen (68,69) bezüglich der Längsachse oder Schwenkebene des Griffteils (30;62) in einem stumpfen Winkel angeordnet sind, wobei der Winkel bevorzugt ca. 110 ° bis ca. 160 ° beträgt.

7. Instrumentarium nach einem der Ansprüche 1 bis 6, **dadurch gekennzeichnet, dass** das Instrument (30) eine Rastvorrichtung (44) aufweist, mittels der die Werkzeugelemente (38,39) in einer oder mehreren vorgegebenen Relativpositionen festlegbar sind.

8. Instrumentarium nach einem der Ansprüche 1 bis 7, **dadurch gekennzeichnet, dass** das Instrument eine Vorrichtung zur Bestimmung des Abstands der Endbereiche der Werkzeugelemente zueinander aufweist.

9. Instrumentarium nach einem der Ansprüche 1 bis 8, **dadurch gekennzeichnet, dass** das Instrument eine Kraftmess-Vorrichtung umfasst, wobei bevorzugt die Kraftmess-Vorrichtung mit einer Anzeige für eine vorgegebene Maximalkraft ausgebildet ist.

10. Instrumentarium nach einem der Ansprüche 1 bis 9, **dadurch gekennzeichnet, dass** das Halteinstrument (160) ein proximal angeordnetes Griffteil (162), einen sich daran anschließenden Schaft (164) und ein am distalen Ende des Schafts angeordnete Haltevorrichtung zum lösbaren Fixieren eines Implantats aufweist; wobei bevorzugt die Haltevorrichtung einen Schlitten (174) aufweist, der vorzugsweise komplementär zu Führungselementen (76,77) des Distraktionsinstruments (60) ausgebildet ist.

11. Instrumentarium nach einem der Ansprüche 1 bis 10, ferner umfassend einen Abstandhalter (80), der bevorzugt ein U-förmig ausgebildetes Abstandselement (82) umfasst; wobei weiter bevorzugt der Abstandhalter (80) als Bohrlehre ausgebildet ist; und wobei optional der Abstandhalter (80) einen Schaft (88) zur extrakorporalen Fixierung aufweist.

12. Instrumentarium nach einem der Ansprüche 1 bis 11, **dadurch gekennzeichnet, dass** das Instrumentarium einen Retraktor (18) umfasst, welcher optional so ausgebildet ist, dass der Abstandhalter (80) mit seinem Schaft (88) an dem Retraktor (18) festlegbar ist.

## Claims

1. Surgical instrumentation for expanding a vertebral canal of a vertebra in the course of a laminoplasty, comprising a distraction instrument (28;60) as well as a holding instrument (160) for an implant to be inserted, wherein during the laminoplasty the vertebral arch of a vertebra is cut through, thereby forming an incision gap, and the incision gap is enlarged with the distraction instrument (28;60) for the purpose of inserting a, in particular, wedge-shaped implant held on the holding instrument (160), wherein proximally the distraction instrument has a gripping part (30;62) and distally two branches (68,69) movable relative to one another with tool elements (38;39) held thereon as well as a lever mechanism arranged between the gripping part (30;62) and the branches (32;68,69), wherein the branches (68,69) comprise guide elements (76,77) which make a guided insertion of the implant into the incision gap possible, wherein the tool elements (38,39;70,71) are held with the lever mechanism so as to be movable relative to one another from a closed rest position into an open spread position, and wherein the tool elements (38,39;70,71) each have a bending strength corresponding to a distraction force of approximately 300 N or more without any plastic deformation occurring at the tool elements (38,39;70,71).

2. Instrumentation as defined in claim 1, **characterized in that** the tool elements (38,39;70,71) have a contact surface (46,47;72,73) at their distal end (40) for the positioning of the instrument (28;60) in the gap (20).

3. Instrumentation as defined in claim 1 or 2, **characterized in that** the tool elements (70,71) have a stop element (74,75) at their distal end for the positioning of an implant in the incision gap (20).

4. Instrumentation as defined in any one of claims 1 to 3, **characterized in that** the tool elements (38,39;70,71) have an end area insertable into the incision gap, the cross section of said end area being of a wedge-shaped design.

5. Instrumentation as defined in any one of claims 1 to 4, **characterized in that** the tool elements (38,39;70,71) are designed so as to taper conically towards their distal end.

6. Instrumentation as defined in any one of claims 1 to 5, **characterized in that** the longitudinal axes of the branches (68,69) holding the tool elements (38,39;70,71) are arranged at an obtuse angle with respect to the longitudinal axis or plane of pivoting of the gripping part (30;62), wherein the angle is approximately 110° to approximately 160°.

7. Instrumentation as defined in any one of claims 1 to 6, **characterized in that** the instrument (30) has a locking device (44) for fixing the tool elements (38,39) in one or more predetermined relative positions.

8. Instrumentation as defined in any one of claims 1 to 7, **characterized in that** the instrument has a device for determining the distance between the end areas of the tool elements.

9. Instrumentation as defined in any one of claims 1 to 8, **characterized in that** the instrument comprises a force measuring device wherein preferably the force measuring device is designed with a display for a predetermined maximum force.

10. Instrumentation as defined in any one of claims 1 to 9, **characterized in that** the holding instrument (160) has a proximally arranged gripping part (162), a shaft (164) adjoining thereto and a holding device arranged at the distal end of the shaft for releasably fixing an implant in position; wherein preferably the holding device has a slide (174) preferably designed complementarily to the guide elements (76,77) of the distraction instrument (60).

11. Instrumentation as defined in any one of claims 1 to 10, further comprising a spacer (80) preferably comprising a spacer element (82) of a U-shaped design; wherein further preferably the spacer (80) is designed as a drilling gauge; and wherein optionally the spacer (80) has a shaft (88) for the extracorporeal fixing in position.

12. Instrumentation as defined in any one of claims 1 to 11, **characterized in that** the instrumentation comprises a retractor (18) optionally designed such that the spacer (80) is adapted to be secured to the retractor (18) with its shaft (88).

## Revendications

1. Ensemble d'instruments chirurgicaux pour élargir un canal rachidien d'une vertèbre dans le cadre d'une laminoplastie, comprenant un instrument de distraction (28 ; 60) ainsi qu'un instrument de maintien (160) pour un implant à insérer, où dans la laminoplastie l'arc rachidien d'une vertèbre est sectionné avec formation d'une fente de coupure et la fente de coupure est élargie avec l'instrument de distraction (28 ; 60) pour l'insertion de l'implant en particulier en forme de coin, maintenu sur l'instrument de maintien (160), où l'instrument de distraction présente en position proximale une partie de préhension (30 ; 62) et en position distale deux branches (68, 69) mobiles l'une par rapport à l'autre avec des éléments d'outil (38 ; 39) maintenus sur celles-ci ainsi qu'un mécanisme de levier disposé entre la partie de préhension (30 ; 62) et les branches (68, 69), où les branches (68, 69) comprennent des éléments de guidage (76, 77) qui permettent une insertion guidée de l'implant dans la fente de coupure, où les éléments d'outil (38, 39 ; 70, 71) sont maintenus de manière mobile l'un par rapport à l'autre avec le mécanisme de levier d'une position de repos fermée dans une position de déploiement ouverte, et où les éléments d'outil (38, 39 ; 70, 71) présentent chacun une résistance en flexion qui correspond à une force de distraction d'environ 300 N ou plus, sans qu'une déformation plastique survienne au niveau des éléments d'outil (38, 39 ; 70, 71).

2. Ensemble d'instruments selon la revendication 1, **caractérisé en ce que** les éléments d'outil (38, 39 ; 70, 71) présentent à leur extrémité distale (40) une surface d'appui (46, 47 ; 72, 73) pour le positionnement de l'instrument (28 ; 60) dans la fente (20).

3. Ensemble d'instruments selon la revendication 1 ou 2, **caractérisé en ce que** les éléments d'outil (70, 71) présentent à leur extrémité distale un élément de butée (74, 75) pour le positionnement d'un implant dans la fente de coupure (20).

4. Ensemble d'instruments selon l'une des revendications 1 à 3, **caractérisé en ce que** les éléments d'outil (38, 39 ; 70, 71) présentent un domaine terminal insérable dans la fente de coupure, dont la section droite est agencée en forme de coin.

5. Ensemble d'instruments selon l'une des revendications 1 à 4, **caractérisé en ce que** les éléments d'outil (38, 39 ; 70, 71) sont agencés de manière rétrécie en forme de cône en direction de leur extrémité distale.

6. Ensemble d'instruments selon l'une des revendications 1 à 5, **caractérisé en ce que** les axes longitudinaux des branches (68, 69) qui maintiennent les éléments d'outil (38, 39 ; 70, 71) sont disposés sous un angle obtus par rapport à l'axe longitudinal ou au plan de pivotement de la partie de préhension (30 ; 62), où l'angle est de préférence d'environ 110° à environ 160° .

7. Ensemble d'instruments selon l'une des revendications 1 à 6, **caractérisé en ce que** l'instrument (30) présente un dispositif à crans (44) au moyen duquel les éléments d'outil (38, 39) peuvent être fixés dans une ou plusieurs positions relatives prédéterminées.

8. Ensemble d'instruments selon l'une des revendications 1 à 7, **caractérisé en ce que** l'instrument présente un dispositif pour la détermination de la distance des domaines terminaux des éléments d'outil entre eux.

9. Ensemble d'instruments selon l'une des revendications 1 à 8, **caractérisé en ce que** l'instrument comprend un dispositif de mesure dynamométrique, où de préférence le dispositif de mesure dynamométrique est agencé avec une indication pour une force maximale prédéterminée.

10. Ensemble d'instruments selon l'une des revendications 1 à 9, **caractérisé en ce que** l'instrument de maintien (160) présente une partie de préhension (162) disposée de manière proximale, une tige (164) qui fait suite à celle-ci et un dispositif de maintien disposé à l'extrémité distale de la tige pour la fixation amovible d'un implant ; où de préférence le dispositif de maintien présente un chariot (174) qui est de préférence agencé de manière complémentaire aux éléments de guidage (76, 77) de l'instrument de distraction (60).

11. Ensemble d'instruments selon l'une des revendications 1 à 10, comprenant en outre un écarteur (80), qui comprend de préférence un élément d'écartement (82) agencé en forme de U ; où de préférence encore l'écarteur (80) est agencé sous forme de gabarit de perçage ; et où éventuellement l'écarteur (80) présente une tige (88) pour la fixation extracorporelle.

12. Ensemble d'instruments selon l'une des revendications 1 à 11, **caractérisé en ce que** l'ensemble d'instruments comprend un rétracteur (18) qui éventuellement est agencé de telle manière que l'écarteur (80) peut être fixé sur le rétracteur (18) avec sa tige (88).
